# EUROPEAN PATENT APPLICATION

(11) **EP 2 301 956 A1**
(43) Date of publication of application: **30.03.2011**
(21) Application number: 09290733.6
(22) Date of filing: 25.09.2009
(51) Int. Cl.: C07K 14/16, C12Q 1/68, G01N 33/569, G01N 33/68

(54) **Methods for prognosticating progression or long term non-progression of a HIV infection or HIV-associated disorders in a patient**

(71) Applicant: Institut Pasteur, 75724 Paris Cedex 15 (FR)
(72) Inventor: Garcia, Alphonse, 92120 Montrouge (FR); Godet, Angélique, 75014 Paris (FR); Croset, Amélie, 74570 Thorens-Glières (FR); Guergnon, Julien, 75014 Paris (FR); Colle, Jean-Hervé, 92340 Bourg-la-Reine (FR); Cayla, Xavier, 37210 Rochecorbon (FR)
(74) Representative: Desaix, Anne

(57) **Abstract**

The present invention relates to a method for analyzing the Vpr protein of a HIV, which method comprises: in a biological sample previously obtained from a patient infected with a HIV, and in particular with a HIV-1, performing an analysis of the nucleotide sequence encoding the tested Vpr protein and/or of the amino acid sequence of the tested Vpr protein, in order to determine the amino acid residue of its sequence which would correspond to the residue at position 84 in the sequence of a reference HIV Vpr protein and/or the amino acid residue of its sequence which would correspond to the residue at position 85 in the sequence of a reference HIV Vpr protein (for example the Vpr protein of the 89.6 HIV-1 isolate).

This method is in particular useful for functionally classifying one or several HIV isolate(s), or for prognosticating, in a patient, progression or long-term non-progression of a HIV infection or of HIV-associated disorders.

SEQUENCE LISTING

<110>  INSTITUT PASTEUR

<120>  METHODS FOR PROGNOSTICATING PROGRESSION OR LONG TERM
       NON-PROGRESSION OF A HIV INFECTION OR HIV-ASSOCIATED DISORDERS IN
        A PATIENT;

<130>  B08427AD/SL/CAL

<160>  45

<170>  PatentIn version 3.3

<210>  1
<211>  16
<212>  PRT
<213>  artificial

<220>
<223>  peptide


<220>
<221>  MISC_FEATURE
<222>  (8)..(8)
<223>  X = X84 = one or several (preferably one) amino acid residue(s),
       which can be, independently, any amino acid residue(s).

<220>
<221>  MISC_FEATURE
<222>  (9)..(9)
<223>  X = X85 = one or several (preferably one) amino acid residue(s),
       which can be, independently, any amino acid residue(s).

<400>  1

Arg His Ser Arg Ile Gly Ile Xaa Xaa Gln Arg Arg Ala Arg Asn Gly
1               5                   10                  15


<210>  2
<211>  16
<212>  PRT
<213>  artificial

<220>
<223>  peptide


<220>
<221>  MISC_FEATURE
<222>  (8)..(8)
<223>  X = X1 = one or several (preferably one) amino acid residue(s),
       which can be, independently, any amino acid residues).

<220>
<221>  MISC_FEATURE
<222>  (9)..(9)
<223>  X = X2 = one or several (preferably one) amino acid residue(s),
       which can be, independently, any amino acid residues).

<400>  2

Arg His Ser Arg Ile Gly Ile Xaa Xaa Gln Arg Arg Ala Arg Asn Gly
1               5                   10                  15

<210>  3
<211>  16
<212>  PRT
<213>  artificial

<220>
<223>  peptide


<220>
<221>  MISC_FEATURE
<222>  (1)..(1)
<223>  X = X77 = one or several (preferably one) amino acid residue(s),
       which can be, independently, any amino acid residues; preferably
       X = X77 = A or R.

<220>
<221>  MISC_FEATURE
<222>  (4)..(4)
<223>  X = X80 = one or several (preferably one) amino acid residue(s),
       which can be, independently, any amino acid residues; preferably
       X = X80 = A or R.

<220>
<221>  MISC_FEATURE
<222>  (13)..(13)
<223>  X = X89 = one or several (preferably one) amino acid residue(s),
       which can be, independently, any amino acid residues; preferably
       X = X89 = A or T.

<400>  3

Xaa His Ser Xaa Ile Gly Ile Ile Gln Gln Arg Arg Xaa Arg Asn Gly
1               5                   10                  15


<210>  4
<211>  12
<212>  PRT
<213>  artificial

<220>
<223>  peptide


<220>
<221>  MISC_FEATURE
<222>  (1)..(1)
<223>  X = X77 = one or several (preferably one) amino acid residue(s),
       which can be, independently, any amino acid residue(s);
       preferably  X = X77 = A or R.

<220>
<221>  MISC_FEATURE
<222>  (4)..(4)
<223>  X = X80 = one or several (preferably one) amino acid residue(s),
       which can be, independently, any amino acid residue(s);
       preferably X = X80 = A or R.

<400>  4

Xaa His Ser Xaa Ile Gly Ile Ile Gln Gln Arg Arg
1               5                   10


<210>  5
<211>  12
<212>  PRT

<213>  artificial

<220>
<223>  peptide


<220>
<221>  MISC_FEATURE
<222>  (2)..(2)
<223>  X = X80 = one or several (preferably one) amino acid residue(s),
       which can be, independently, any amino acid residue(s);
       preferably X = X80 = A or R.

<220>
<221>  MISC_FEATURE
<222>  (11)..(11)
<223>  X = X89 = one or several (preferably one) amino acid residue(s),
       which can be, independently, any amino acid residue(s):
       preferably X = X89 = A or T.

<400>  5

Ser Xaa Ile Gly Ile Ile Gln Gln Arg Arg Xaa Arg
1               5                   10


<210>  6
<211>  12
<212>  PRT
<213>  artificial

<220>
<223>  peptide


<220>
<221>  MISC_FEATURE
<222>  (9)..(9)
<223>  X = X89 = one or several (preferably one) amino acid residue(s),
       which can be, independently, any amino acid residue(s);
       preferably X = X89 = A or T.

<400>  6

Ile Gly Ile Ile Gln Gln Arg Arg Xaa Arg Asn Gly
1               5                   10


<210>  7
<211>  10
<212>  PRT
<213>  artificial

<220>
<223>  peptide


<220>
<221>  MISC_FEATURE
<222>  (1)..(1)
<223>  X = X80 = one or several (preferably one) amino acid residue(s),
       which can be, independently, any amino acid residue(s);
       preferably X = X80 = A or R.

<220>
<221>  MISC_FEATURE
<222>  (10)..(10)
<223>  X = X89 = one or several (preferably one) amino acid residue(s),

which can be, independently, any amino acid residue(s);
preferably X = X89 = A or T.

<400> 7

Xaa Ile Gly Ile Ile Gln Gln Arg Arg Xaa
1               5                   10


<210> 8
<211> 9
<212> PRT
<213> artificial

<220>
<223> peptide


<220>
<221> MISC_FEATURE
<222> (1)..(1)
<223> X = X80 = one or several (preferably one) amino acid residue(s),
which can be, independently, any amino acid residue(s),
preferably X = 80 = A or R.

<400> 8

Xaa Ile Gly Ile Ile Gln Gln Arg Arg
1               5


<210> 9
<211> 9
<212> PRT
<213> artificial

<220>
<223> peptide


<220>
<221> MISC_FEATURE
<222> (9)..(9)
<223> X = X89 = one or several (preferably one) amino acid residue(s),
which can be, independently, any amino acid residue(s);
preferably X = X89 = A or T.

<400> 9

Ile Gly Ile Ile Gln Gln Arg Arg Xaa
1               5


<210> 10
<211> 8
<212> PRT
<213> artificial

<220>
<223> peptide

<400> 10

Ile Gly Ile Ile Gln Gln Arg Arg
1               5


<210> 11

<211>  16
<212>  PRT
<213>  artificial

<220>
<223>  peptide

<400>  11

Arg His Ser Arg Ile Gly Ile Ile Gln Gln Arg Arg Thr Arg Asn Gly
1               5                   10                  15


<210>  12
<211>  12
<212>  PRT
<213>  artificial

<220>
<223>  peptide

<400>  12

Arg His Ser Arg Ile Gly Ile Ile Gln Gln Arg Arg
1               5                   10


<210>  13
<211>  12
<212>  PRT
<213>  artificial

<220>
<223>  peptide

<400>  13

Ser Arg Ile Gly Ile Ile Gln Gln Arg Arg Thr Arg
1               5                   10


<210>  14
<211>  12
<212>  PRT
<213>  artificial

<220>
<223>  peptide

<400>  14

Ile Gly Ile Ile Gln Gln Arg Arg Thr Arg Asn Gly
1               5                   10


<210>  15
<211>  16
<212>  PRT
<213>  artificial

<220>
<223>  peptide

<400>  15

Ala His Ser Ala Ile Gly Ile Ile Gln Gln Arg Arg Thr Arg Asn Gly
1               5                   10                  15

<210>  16
<211>  16
<212>  PRT
<213>  artificial

<220>
<223>  peptide

<400>  16

Arg His Ser Ala Ile Gly Ile Ile Gln Gln Arg Arg Thr Arg Asn Gly
1               5                   10                  15


<210>  17
<211>  16
<212>  PRT
<213>  artificial

<220>
<223>  peptide

<400>  17

Ala His Ser Arg Ile Gly Ile Ile Gln Gln Arg Arg Thr Arg Asn Gly
1               5                   10                  15


<210>  18
<211>  16
<212>  PRT
<213>  artificial

<220>
<223>  peptide

<400>  18

Ala His Ser Ala Ile Gly Ile Ile Gln Gln Arg Arg Ala Arg Asn Gly
1               5                   10                  15


<210>  19
<211>  16
<212>  PRT
<213>  artificial

<220>
<223>  peptide

<400>  19

Arg His Ser Ala Ile Gly Ile Ile Gln Gln Arg Arg Ala Arg Asn Gly
1               5                   10                  15


<210>  20
<211>  16
<212>  PRT
<213>  artificial

<220>
<223>  peptide

<400>  20

Ala His Ser Arg Ile Gly Ile Ile Gln Gln Arg Arg Ala Arg Asn Gly

1               5                   10                  15


<210>  21
<211>  16
<212>  PRT
<213>  artificial

<220>
<223>  peptide

<400>  21

Arg His Ser Arg Ile Gly Ile Ile Gln Gln Arg Arg Ala Arg Asn Gly
1               5                   10                  15


<210>  22
<211>  12
<212>  PRT
<213>  artificial

<220>
<223>  peptide

<400>  22

Ala His Ser Ala Ile Gly Ile Ile Gln Gln Arg Arg
1               5                   10


<210>  23
<211>  12
<212>  PRT
<213>  artificial

<220>
<223>  peptide

<400>  23

Ala His Ser Arg Ile Gly Ile Ile Gln Gln Arg Arg
1               5                   10


<210>  24
<211>  12
<212>  PRT
<213>  artificial

<220>
<223>  peptide

<400>  24

Arg His Ser Ala Ile Gly Ile Ile Gln Gln Arg Arg
1               5                   10


<210>  25
<211>  12
<212>  PRT
<213>  artificial

<220>
<223>  peptide

<400>  25

Ser Ala Ile Gly Ile Ile Gln Gln Arg Arg Ala Arg
1               5                   10

<210>  26
<211>  12
<212>  PRT
<213>  artificial

<220>
<223>  peptide

<400>  26

Ser Ala Ile Gly Ile Ile Gln Gln Arg Arg Thr Arg
1               5                   10

<210>  27
<211>  12
<212>  PRT
<213>  artificial

<220>
<223>  peptide

<400>  27

Ser Arg Ile Gly Ile Ile Gln Gln Arg Arg Ala Arg
1               5                   10

<210>  28
<211>  12
<212>  PRT
<213>  artificial

<220>
<223>  peptide

<400>  28

Ile Gly Ile Ile Gln Gln Arg Arg Ala Arg Asn Gly
1               5                   10

<210>  29
<211>  10
<212>  PRT
<213>  artificial

<220>
<223>  peptide

<400>  29

Ala Ile Gly Ile Ile Gln Gln Arg Arg Ala
1               5                   10

<210>  30
<211>  10
<212>  PRT
<213>  artificial

<220>
<223>  peptide

<400>  30

Ala Ile Gly Ile Ile Gln Gln Arg Arg Thr
1               5                   10


<210>  31
<211>  10
<212>  PRT
<213>  artificial

<220>
<223>  peptide

<400>  31

Arg Ile Gly Ile Ile Gln Gln Arg Arg Ala
1               5                   10


<210>  32
<211>  9
<212>  PRT
<213>  artificial

<220>
<223>  peptide

<400>  32

Ala Ile Gly Ile Ile Gln Gln Arg Arg
1               5


<210>  33
<211>  9
<212>  PRT
<213>  artificial

<220>
<223>  peptide

<400>  33

Ile Gly Ile Ile Gln Gln Arg Arg Ala
1               5


<210>  34
<211>  10
<212>  PRT
<213>  artificial

<220>
<223>  peptide

<400>  34

Arg Ile Gly Ile Ile Gln Gln Arg Arg Thr
1               5                   10


<210>  35
<211>  9
<212>  PRT
<213>  artificial

<220>
<223>  peptide

<400>  35

Arg Ile Gly Ile Ile Gln Gln Arg Arg
1               5

<210>  36
<211>  9
<212>  PRT
<213>  artificial

<220>
<223>  peptide

<400>  36

Ile Gly Ile Ile Gln Gln Arg Arg Thr
1               5

<210>  37
<211>  12
<212>  PRT
<213>  artificial

<220>
<223>  peptide

<400>  37

His Phe Arg Ile Gly Cys Arg His Ser Arg Ile Gly
1               5                   10

<210>  38
<211>  16
<212>  PRT
<213>  artificial

<220>
<223>  peptide

<400>  38

Arg His Ser Arg Ile Gly Val Thr Arg Gln Arg Arg Ala Arg Asn Gly
1               5                   10                  15

<210>  39
<211>  16
<212>  PRT
<213>  artificial

<220>
<223>  peptide

<400>  39

Arg His Ser Arg Ile Gly Ile Pro Gln Gln Arg Arg Ala Arg Asn Gly
1               5                   10                  15

<210>  40
<211>  26
<212>  PRT

<213>  artificial

<220>
<223>  peptide

<400>  40

His Phe Arg Ile Gly Cys Arg His Ser Arg Ile Gly Val Thr Arg Gln
1               5                   10                  15

Arg Arg Ala Arg Asn Gly Ala Ser Arg Ser
            20                  25

<210>  41
<211>  96
<212>  PRT
<213>  Artificial

<220>
<223>  peptide

<400>  41

Met Glu Gln Ala Pro Glu Asp Gln Gly Pro Gln Arg Glu Pro Tyr Asn
1               5                   10                  15

Asp Trp Thr Leu Glu Leu Leu Glu Glu Leu Lys Asn Glu Ala Val Arg
            20                  25                  30

His Phe Pro Arg Ile Trp Leu His Ser Leu Gly Gln His Ile Tyr Glu
        35                  40                  45

Thr Tyr Gly Asp Thr Trp Thr Gly Val Glu Ala Leu Ile Arg Ile Leu
    50                  55                  60

Gln Gln Leu Leu Phe Ile His Phe Arg Ile Gly Cys Arg His Ser Arg
65                  70                  75                  80

Ile Gly Ile Ile Gln Gln Arg Arg Thr Arg Asn Gly Ala Ser Lys Ser
                85                  90                  95

<210>  42
<211>  96
<212>  PRT
<213>  Artificial

<220>
<223>  PEPTIDE

<400>  42

Met Glu Gln Ala Pro Glu Asp Gln Gly Pro Gln Arg Glu Pro Tyr Asn
1               5                   10                  15

Asp Trp Thr Leu Glu Leu Leu Glu Glu Leu Lys Asn Glu Ala Val Arg
            20                  25                  30

His Phe Pro Arg Ile Trp Leu His Ser Leu Gly Gln His Ile Tyr Glu

35                  40                  45

Thr Tyr Gly Asp Thr Trp Thr Gly Val Glu Ala Leu Ile Arg Ile Leu
    50                  55                  60

Gln Gln Leu Leu Phe Ile His Phe Arg Ile Gly Cys Arg His Ser Arg
65                  70                  75                  80

Ile Gly Ile Ile Gln Gln Arg Arg Ala Arg Asn Gly Thr Ser Arg Ser
                85                  90                  95

<210>  43
<211>  96
<212>  PRT
<213>  Artificial

<220>
<223>  peptide

<400>  43

Met Glu Gln Ala Pro Glu Asp Gln Gly Pro Gln Arg Glu Pro Tyr Asn
1               5                   10                  15

Asp Trp Thr Leu Glu Leu Leu Glu Glu Leu Lys Asn Glu Ala Val Arg
            20                  25                  30

His Phe Pro Arg Ile Trp Leu His Ser Leu Gly Gln His Ile Tyr Glu
        35                  40                  45

Thr Tyr Gly Asp Thr Trp Thr Gly Val Glu Ala Leu Ile Arg Ile Leu
    50                  55                  60

Gln Gln Leu Leu Phe Ile His Phe Arg Ile Gly Cys Arg His Ser Arg
65                  70                  75                  80

Ile Gly Val Thr Arg Gln Arg Arg Ala Arg Asn Gly Ala Ser Arg Ser
                85                  90                  95

<210>  44
<211>  16
<212>  PRT
<213>  Artificial

<220>
<223>  peptide

<220>
<221>  MISC_FEATURE
<222>  (8)..(8)
<223>  X = X84 = one or several (preferably one) amino acid residue(s),
       which can be, independently, any amino acid residue(s)

<220>
<221>  MISC_FEATURE
<222>  (9)..(9)
<223>  X = X85 = one or several (preferably one) amino acid residue(s),

which can be, independently, any amino acid residue(s)

<400>  44

Arg His Ser Arg Ile Gly Ile Xaa Xaa Gln Arg Arg Thr Arg Asn Gly
1               5                   10                  15

<210>  45
<211>  16
<212>  PRT
<213>  Artificial

<220>
<223>  peptide

<220>
<221>  MISC_FEATURE
<222>  (8)..(8)
<223>  X = X1 = one or several (preferably one) amino acid residue(s),
       which can be, independently, any amino acid residue(s).

<220>
<221>  MISC_FEATURE
<222>  (9)..(9)
<223>  X = X2 = one or several amino acid residue(s), (preferably one
       residue), which can be, independently, any amino acid residue(s).

<400>  45

Arg His Ser Arg Ile Gly Ile Xaa Xaa Gln Arg Arg Thr Arg Asn Gly
1               5                   10                  15

## Description

The present invention relates to a method for analyzing the Vpr protein of a Human Immunodeficiency Virus (HIV), in particular a human immunodeficiency virus type 1 (HIV-1).

This method can be used for several purposes, and in particular for functionally classifying one or several HIV(s), or for prognosticating, in a patient, progression or long-term non progression of a HIV infection or of HIV-associated disorders, and in particular AIDS progression and neuro-AIDS genesis, and especially of HIV associated dementia (HAD) and tauopathies.

The invention also relates to novel synthetic or natural peptides derived from the C-terminal region of the Vpr protein of a HIV, and in particular from the region of the Vpr protein of a HIV which would correspond to the one ranging from amino acid residues 77 to 92 in the sequence of a reference HIV Vpr protein (for example the Vpr protein of the 89.6 HIV-1 isolate). These peptides, which are less than 30 amino acids in size, are able to bind a type 2A protein phosphatase holoenzyme or one of its subunits *in vitro.* They can be used in particular for delivering one or several compounds in particular one or several polypeptides into a cell, for inducing or increasing apoptosis of tumor cells or tumor cell lines, especially human tumor cells or cell lines and more generally human cells.

HIV infection provokes the depletion of CD4+ T cells in HIV positive patients. This depletion is a consequence of the activation of multiple death pathways in both infected cells and non-infected "bystander" cells¹. HIV-1 Vpr is one of the auxiliary HIV-1 proteins that are important for *in vivo* establishment and/or maintenance of AIDS pathogenesis. Multiple studies indicate that HIV-1 Vpr regulates viral replication *in vivo* and is required for virus replication in non-dividing cells. In addition, HIV-1 Vpr induces cell cycle arrest in proliferative cells, stimulates virus transcription and can induce apoptosis of infected cells². Previous studies also suggested that Vpr can induce apoptosis in distinct human cells as a consequence of the prolonged cell cycle arrest³.

Furthermore, recent reports have clearly documented a major apoptotic mechanism which is based on the physical interaction of the HIV-1 Vpr protein with the Adenine Nucleotide Translocator, a component of the permeability transition pore of mitochondria localized in the inner mitochondrial membrane^{5;6}. This mitochondriotoxic domain corresponds to the residues 71-82 that are partially located at the end of the third α-helix of the HIV-1 Vpr protein (Vpr₅₆₋₇₇). The sequence of this domain is highly conserved between different HIV isolates.

Protein phosphatase type 2A (PP2A) holoenzyme represents a major family of serine/threonine protein phosphatases that has been implicated in the regulation of many cellular processes, including cell growth and apoptosis in mammalian cells⁷. PP2A proteins exist *in vivo* in two classes with different forms: a dimeric form (AC) consisting of a catalytic C subunit (PP2Ac) and a structural A subunit, and a trimeric form (ABC). Subunit (B) can regulate both the substrate specificity and localization of the trimeric holoenzyme⁸. PP2A₁ is a trimeric form composed of A, Bα and C subunit.

We previously reported experimental evidence providing proof of principle for the Drug Phosphatase Technology (DPT), a novel concept for rational drug design based on the identification and the use of penetrating peptide sequences that interact with PP1/PP2A holoenzymes to regulate cell death⁹.

Given the major role of PP2A holoenzymes in virus-host interactions, the importance of identifying the binding sites of HIV Vpr proteins with PP2A holoenzymes or one of their subunits can be understood, so that novel therapeutic targets can be identified.

In particular, the identification of Vpr-derived peptides or polypeptides interacting with PP2A holoenzyme should allow novel drugs to be developed that can block, by competitive inhibition, the cell mechanisms induced by HIV-Vpr proteins via their interaction with PP2A holoenzyme.

The absence of common motifs to the series of proteins interacting with PP2A holoenzyme prevented the computer-based identification of peptide motifs directly involved in binding those proteins with PP2A holoenzyme.

This study builds upon our previous biochemical identification of the Vpr₇₇₋₉₂ domain, located in the C-terminal portion of the HIV-1 Vpr protein from 89.6 HIV-1 isolate as a PP2A₁ binding domain⁴.

The results presented in the example part of the present application suggest in particular that this Vpr₇₇₋₉₂ domain, and in particular, the amino acid residues which are located at positions 84 and 85 in the Vpr protein, are involved in pathways favoring development of HIV infection towards acquired immunodeficiency syndrome (AIDS) and/or in neuro-AIDS genesis.

Hence, a first aspect of the invention is a method for analyzing the Vpr protein of a HIV, in particular a HIV-1, which Vpr protein is termed "tested Vpr protein". This method comprises or consists of: in a biological sample previously obtained from a patient infected with a HIV, and in particular with a HIV-1, performing an analysis of the nucleotide sequence encoding the tested Vpr protein and/or of the amino acid sequence of the tested Vpr protein, in order to determine the amino acid residue of its sequence which is termed X₈₄ and/or the amino acid residue of its sequence which is termed X₈₅, which residues would respectively correspond to the residues at position 84 and position 85 in the sequence of a reference HIV Vpr protein, and in the context of said sequence.

In other words, the invention relates to the detection of amino acid residues of the tested Vpr protein which are at positions 84 and 85 in said sequence when aligned with the sequence of a reference Vpr-protein.

Although alignment of sequences is referred to, it is not necessary in practice. The targeted positions may indeed be determined from sequences which have a number of amino acid residues sufficient to identify the region encompassing residues at positions 84 and 85, such as 5 to 20 or 5 to 15 residues naturally surrounding said positions.

This method, as well as any other method disclosed herein, is performed in *vitro* and/or *ex vivo.*

Alternatively or cumulatively, the method for analyzing a tested Vpr protein according to the invention can comprise or consist of: in a biological sample previously obtained from a patient infected with a HIV, and in particular with a HIV-1, performing an analysis of the nucleotide sequence encoding the tested Vpr protein and/or of the amino acid sequence of the tested Vpr protein, in order to detect the presence, in the C-terminal region of said tested Vpr protein, which comprises all or part of the sequence of said tested Vpr protein which would correspond to the one ranging from amino acid residues 77 to 92 in the sequence of a reference HIV Vpr protein, of an amino acid sequence comprising or consisting of sequence isoleucine-glutamine (IQ) or of a sequence chosen from the following group: IQ, TR, IR, LR, IP, LQ, TP, MR, VR, LP, SL and QQ, and in particular from the following group: IQ, TR, IR, LR, IP, LQ, TP, MR, VR and LP.

Alternatively or cumulatively, the method for analyzing a tested Vpr protein according to the invention can comprise or consist of: in a biological sample previously obtained from a patient infected with a HIV, and in particular with a HIV-1, performing an analysis of the nucleotide sequence encoding the tested Vpr protein and/or of the amino acid sequence of the tested Vpr protein, in order to determine the amino acid residues X₈₄ and X₈₅ in a peptide sequence chosen from the following group:
RHSRIGIX₈₄X₈₅QRRARNG (SEQ ID No: 1); or
RHSRIGIX₈₄X₈₅QRRTRNG (SEQ ID No: 44),
or in a variant of said sequence derived therefrom by substitution of 1 to 5 amino acid residue(s) and present in said tested Vpr protein, wherein X₈₄ and X₈₅ independently designate one or several amino acid residue(s), which can be, independently, any amino acid residue(s).

Unless otherwise specified, any embodiment disclosed herein can be used independently and/or in combination with the others embodiments.

In a particular embodiment of the invention, the terms X₈₄ and X₈₅ as used herein independently designate a single amino acid residue, corresponding to the residues at positions 84 and 85 respectively in a Vpr protein whose sequence would be aligned with the sequence of a reference Vpr protein.

In a particular embodiment of the invention, residues X₈₄ and X₈₅ as defined and used herein correspond to amino acids residues number 84 and 85 in the sequence of the tested Vpr protein.

As used herein, the term "HIV" designates a Human Immunodeficiency Virus. In a particular embodiment of the invention, this term designates a type or subtype of HIV. In particular, a "HIV" can be a HIV-1, or a subtype of HIV-1.

Similarly, a "HIV-1" as used herein designates a strain of HIV-1 virus and can designate in particular a subtype of HIV-1.

By "HIV isolate", it is meant herein a HIV, in particular a HIV-1, which has been isolated from a biological sample obtained from a HIV infected patient.

In a particular embodiment of the invention, the "reference Vpr protein" as used herein is the Vpr protein of the 89.6 isolate of HIV-1 ²⁵, or, more preferably, the Vpr protein of the Lai isolate of HIV-1 which has amino acid sequence disclosed under accession number GenBank Z68546...

As used herein, the term "tested Vpr protein" can designate a native Vpr protein (*i.e.,* a full-length Vpr protein) and/or cleavage product(s) of this native Vpr protein. In a preferred embodiment of the invention, these cleavage products comprise residue X₈₄ and/or residue X₈₅ of the native Vpr protein, and more preferably both residues X₈₄ and X₈₅ of the native Vpr protein, as a sequence X₈₄ X₈₅ of said Vpr protein, in the context of their naturally adjacent residues.

A "biological sample" as used herein can be a sample of cells, in particular a sample of cultured cells, or a sample of body fluid (for example blood, plasma or cephalorachidian liquid (CRL)), or a sample of tissue. Said sample can be derived from any living organism which is susceptible to infection by an immunodeficiency virus, in particular a HIV, preferably a mammal, and more preferably a patient (*i.e.,* a human). Said sample can be in particular derived from a HIV-positive patient. It can be for example a sample of CD4 cells or a sample of plasma or CRL, which is derived from a patient infected with a VIH, and in particular with a VIH-1.

In a particular embodiment of the invention, the "biological sample" comprises at least one HIV-Vpr protein or a nucleic acid encoding the same. Vpr proteins of different HIV, and in particular different HIV types or sub-types, or nucleic acids encoding the same can be present in a biological sample. This can be the case for example when a patient has been infected with different HIV (for example two different HIV), and in particular with HIV of different types or sub-types, for example with two or more than two HIV-1 of different sub-types.

In a particular embodiment of the invention, the biological sample comprises the Vpr protein of each HIV, and in particular each HIV type or sub-type, which has infected the mammal (in particular the human patient) from which the biological sample was obtained, or a nucleic acid encoding said Vpr protein(s).

In a particular embodiment of the invention, the method for analyzing a tested Vpr protein of the invention requires either analyzing its amino acid sequence or, preferably, analyzing the nucleotide sequence encoding its amino acid sequence, said nucleic acid being present in the sample as viral RNA or viral DNA.

In a particular embodiment of the invention, the sequence analysis (*i.e.,* the nucleotide sequence analysis and/or the amino acid sequence analysis) of the tested Vpr protein is performed in order to determine:
- whether X₈₄ consists of an amino acid residue chosen from the following group: I, L, T, M and V, preferably from the following group: I, L and T; and/or
- whether X₈₅ consists of an amino acid residue chosen from the following group: Q, T, R and P, and preferably from the following group: Q, T and R.

In a particular embodiment of the invention, the analysis of the nucleotide sequence and/or of the amino acid sequence is performed in order to determine both X₈₄ and X₈₅ residues.

In a particular embodiment of the invention, the sequence analysis is performed in order to determine whether amino acid sequence X₈₄X₈₅ consists of sequence IQ or of a peptide sequence chosen from the following group: IQ, TR, IR, LR, IP, LQ, TP, MR, VR, LP, SL and QQ, and in particular from the following group: IQ, TR, IR, LR, IP, LQ, TP, MR, VR and LP. Hence, the sequence analysis is performed in order to determine whether X₈₄X₈₅ is TR and/or whether X₈₄X₈₅ is IR and/or whether X₈₄X₈₅ is LR and/or whether X₈₄X₈₅ is IP and/or whether X₈₄X₈₅ is LQ and/or whether X₈₄X₈₅ is TP and/or whether X₈₄X₈₅ is MR and/or whether X₈₄X₈₅ is VR and/or whether X₈₄X₈₅ is LP and/or whether X₈₄X₈₅ is SL and/or whether X₈₄X₈₅ is QQ.

In a particular embodiment, the sequence analysis can be performed in order to determine whether X₈₄X₈₅ is IQ, whether X₈₄X₈₅ is TR, whether X₈₄X₈₅ is IR, whether X₈₄X₈₅ is LR, whether X₈₄X₈₅ is IP, whether X₈₄X₈₅ is LQ, whether X₈₄X₈₅ is TP, whether X₈₄X₈₅ is MR, whether X₈₄X₈₅ is VR, and whether X₈₄X₈₅ is LP.

The different nucleotide or amino acid sequence analyses which are required in order to determine X₈₄ and/or X₈₅ can be performed simultaneously or separately but they are preferably performed simultaneously.

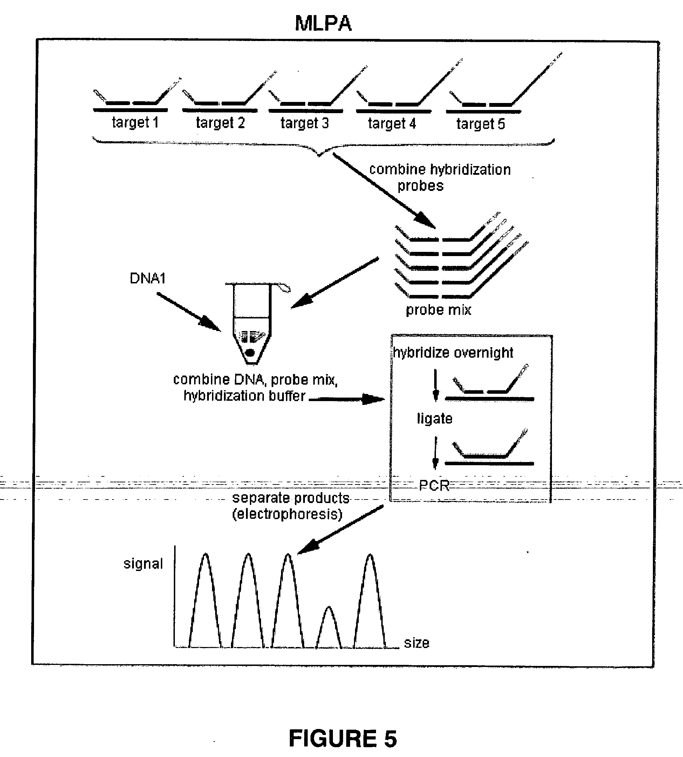

In a particular embodiment of the invention, the analysis of the nucleotide sequence encoding the tested Vpr protein is performed by PCR, in particular by PCR multiplex ligation probe assay (MLPA) and/or by ELISA assay. The principle of MLPA is illustrated in figure 5.

The analysis of the nucleotide sequence encoding the tested Vpr protein can be performed by PCR, using specific primers chosen to specifically amplify variable sequences of the domain Vpr₇₇₋₉₂.

In a further aspect, the invention relates to a method for analyzing one or several HIV(s), in particular one or several HIV-1 isolate(s), said method comprising analyzing the Vpr protein of said HIV(s) by the method of analysis of the invention. Said method can be useful for example for typing one or several HIV(s) and especially for establishing a new functional classification of HIV variants and in particular of HIV-1(s), infecting a patient.

Hence, the present invention is directed to a method for functionally classifying one or several HIV or HIV-1, in particular one or several HIV or HIV-1 (s), said method comprising analyzing the Vpr protein of said HIV(s) by the method of analysis of the invention.

Among the parameter enabling to functionally classify HIV (in particular HIV isolates), the finding that in the Vpr protein of a given HIV, amino acid sequence X₈₄X₈₅ is IQ is indicative that the HIV may be classified in a category of HIVs that may induce genesis or progression of AIDS and/or neuro-AIDS in a patient. This means in particular that in a patient infected with such a HIV, progression of HIV infection, and especially genesis or progression of AIDS and/or neuro-AIDS may be expected. For the sake of convenience, such a virus will be termed herein highly virulent virus.

On the contrary, the finding that in the Vpr protein of a given HIV, X₈₄ is not amino acid residue I and/or that X₈₅ is not amino acid residue Q, and in particular the finding that X₈₄X₈₅ is a peptide sequence chosen from the following group: TR, IR, LR, IP, LQ, TP, MR, VR, LP, SL and QQ, or from the following group: TR, IR, LR, IP, LQ, TP, MR, VR and LP, is indicative that the HIV may be classified in a category of HIVs that may not induce AIDS and/or neuro-AIDS in a patient. For the sake of convenience, such an HIV will be termed herein "less virulent virus".

The observed difference(s) between so-called amino acid residues X₈₄ X₈₅ in the tested Vpr protein of HIVs provides an element for a functional classification of HIV as it provides information on the possible evolution of the infection toward AIDS or its various stages.

In a patient infected with a less virulent virus according to the proposed categories of the invention, a long-term non progression may be expected, provided that said patient is not also infected with highly virulent virus.

In case a patient has been infected with both highly and less virulentviruses, the highly virulent phenotype should be dominant. This means that in a patient infected with both highly virulent and virulent viruses, progression of HIV infection, and especially genesis or progression of AIDS and/or neuro-AIDS in said is predictable.

By "neuro-AIDS", it is meant herein HIV-associated neuropathologies, and in particular HIV-associated dementia (HAD) or neurodegenerative pathologies, for example tauopathies.

In another aspect, the invention also relates to a method for providing prognosticating elements, in relation to *in vitro* and/or *ex vivo,* progression or long-term non-progression of a HIV infection or of HIV-associated disorders in a patient, and in particular for providing prognosticating elements in relation to *in vitro* and/or *ex vivo,* genesis or progression of AIDS and/or HIV-associated disorders in particular neuro-AIDS. Said method comprises the following steps:
a) analyzing, by the method of analysis of the invention, one or several HIV-Vpr protein(s) which is (are) present in a biological sample previously obtained from a patient infected with at least one HIV, in particular at least one HIV-1; and
b) using the results obtained in step a) in a process for determining possible progression or long-term non-progression of HIV infection or HIV-associated disorders in said patient.

In a particular embodiment of this prognosis method of the invention, the finding that in a protein present in the biological sample, X₈₄ is not amino acid residue I and/or that X₈₅ is not amino acid residue Q, and in particular the finding that in a Vpr protein present in the biological sample, X₈₄X₈₅ is a peptide sequence chosen from TR, IR, LR, IP, LQ, TP, MR, VR, LP, SL and QQ, and in particular from TR, IR, LR, IP, LQ, TP, MR, VR, LP, SL and QQ or from TR, IR, TP, MR, SL and QQ, is indicative that a long-term non progression of HIV infection or HIV-associated disorders in the patient may be expected.

On the contrary, in a particular embodiment of this prognosis method of the invention, the finding that in a Vpr protein present in the biological sample, amino acid sequence X₈₄X₈₅ is IQ is indicative that a progression of HIV infection or HIV-associated disorders in the patient may be expected, and in particular that genesis or progression of AIDS and/or of neuro-AIDS in the patient may be expected.

In a further aspect, the invention relates to another method for providing prognosticating elements, in relation to *in vitro* and/or *ex vivo,* progression or long-term non-progression of a HIV infection or of HIV-associated disorders in a patient and in particular for providing prognosticating elements in relation to *in vitro* and/or *ex vivo,* genesis or progression of AIDS and/or neuro-AIDS. Said method comprises or consists of the following steps:
a) determining whether a biological sample previously obtained from a patient infected with at least one HIV, in particular at least one HIV-1, contains at least one HIV Vpr protein which is able to bind a PP2A holoenzyme (in particular PP2A₁) or one of its subunits, or which is able to induce or increase apoptosis of tumor cells or tumor cell lines, especially human tumor cells or cell lines" and
b) using the results obtained in step a) in a process for determining possible progression or long-term non-progression of HIV infection or HIV-associated disorders, wherein
   - the presence, in the biological sample, of at least one Vpr protein having said ability is indicative that a progression of HIV infection or HIV-associated disorders in the patient may be expected, and in particular that genesis or progression of AIDS and/or of neuro-AIDS, whereas
   - the absence, in the biological sample, of any Vpr protein having said ability is indicative that a long-term non progression of HIV infection or HIV-associated disorders in the patient may be expected.

Step a) can be performed by analyzing directly the HIV Vpr protein(s) present in said sample or by analyzing peptides or polypeptides derived from the HIV Vpr protein(s) present(s) in said sample, which peptides or polypeptides comprise amino acid residues X₈₄ and X₈₅ as defined herein of the HIV Vpr protein.

In a particular embodiment of the invention, a "peptide" as used herein comprises or consists of at least 2, preferably at least 4, and more preferably at least 6 or 8 consecutive amino acid residues, and/or consists of less than 30 and preferably less than 20 consecutive amino acid residues, for example less than 17 or 15 consecutive amino acid residues. For example, a "peptide" as used herein can consist of 6 to 20 and preferably 8 to 16 amino acid residues, for example, 6, 7, 8, 9, 10, 11, 12, 13, 14, 15, or 16 consecutive amino acid residues.

In a particular embodiment of the invention, a "polypeptide" as used herein consists of: at least 30 consecutive amino acid residues, and/or less than 90, and preferably less 80 or 70 consecutive amino acid residues. For example, a "polypeptide" as used herein can consist of 30 to 90 and preferably 35 to 80 or 30 to 50 consecutive amino acid residues.

In the present invention, the term "at least x" means x or more than x. Hence, "at least one" means one or more than one (*i.e.,* one or several).

In a particular embodiment of the invention, the analyzed Vpr-derived peptides or polypeptides are derived from the region of the Vpr protein which would correspond to the one ranging from amino acid residues 77 to 92 in the sequence of a reference HIV Vpr protein (for example the Vpr protein of the 89.6 HIV-1 isolate).

In a particular embodiment of the invention, Vpr-derived peptides or polypeptides as used herein are derived from a polypeptidic region having sequence chosen from the following group :
RHSRIGIX₁X₂QRRARNG (SEQ ID No: 2), and
RHSRIGIX₁X₂QRRTRNG (SEQ ID No: 45),
wherein X₁ and X₂ independently designate one or several amino acid residue(s), preferably a single amino acid residue, which can be, independently, any amino acid residue(s).

By "peptides or polypeptides derived from region x", it is meant herein peptides or polypeptides that consist of or comprise the sequence of said region x or a sequence which differs from the original amino acid sequence of said region x by insertion, substitution or deletion (preferably substitution or deletion, and more preferably deletion) of one or several amino acid residues (for example 1, 2, 3, 4 or 5 amino acid residues) in the original amino acid sequence. A substitution of amino acid residues can be either conservative, semi-conservative or non-conservative.

In a particular embodiment of the invention, the peptides or polypeptides "derived from region x" comprise or consist of a sequence which is at least 70, 80% or 90% identical or similar to the peptide sequence of region x.

The term "type 2A protein phosphatase holoenzyme" (or PP2A holoenzyme) as used herein means any purified dimeric (AC) or heterotrimeric (ABC) complex of a cellular or reconstituted extract after purifying two subunits (A) and (C) of a PP2A holoenzyme and, if necessary, a subunit (B). The type PP2A holoenzymes are preferably derived from mammals. An example of PP2A is PP2A₁.

By "subunit", it is meant in particular subunit (A), (B) or (C).

In a particular embodiment of the invention, by "bind(s) a PP2A holoenzyme or one of its subunits", it is meant herein bind(s) *in vitro* a PP2A holoenzyme or one of its subunits.

In a particular embodiment of the invention, a Vpr protein or a Vpr-derived peptide or polypeptide which is able to bind a PP2A holoenzyme or one of its subunits, specifically binds a PP2A holoenzyme or one of its subunits, which means that it is capable of competitively inhibiting binding of a HIV-Vpr protein or of a HIV-Vpr derived peptide or polypeptide with one or several PP2A holoenzymes or one or several subunits thereof.

The Vpr protein(s), or Vpr-derived peptide(s) or polypeptide(s) onto which the PP2A holoenzyme is bound is generally identified by direct or indirect labeling of the protein phosphatase. Binding of PP2A holoenzyme or one of its subunits to a Vpr protein or to a Vpr-derived peptide or polypeptide can then be revealed, in particular using antiserums, using techniques that are conventionally used for Western Blot or solid phase ELISA test, after incubating a support containing the Vpr protein(s), or Vpr-derived peptide(s) or polypeptide(s) of the biological sample with an antibody directed against subunits (A) or (B) or (C) or a mixture of antibodies directed against subunits (A), (B) or (C) of PP2A holoenzyme

The ability to induce or increase apoptosis of tumor cells or tumor cell lines, especially human tumor cells or cell lines can be assessed as disclosed herein or by any other method.

In another aspect, the invention also pertains to a peptide of reduced size, which is capable of binding a PP2A holoenzyme (in particular PP2A₁) or one of its subunits. In contrast to native proteins or polypeptide domains of large size, reduced size peptides have the advantage of being readily synthesized, either chemically or in cell systems, in high yields and cheaply. The peptide of the invention is also more stable and more readily transferred into the cytoplasm or into the nucleus of cells using, if required, appropriate vectors, with a view to therapeutic use.

The peptide of the invention is a natural or synthetic peptide of less than 30 amino acids in size, preferably less than 20 amino acids.

One peptide of the invention is derived from the C-terminal region of a Vpr protein of a HIV, for example HIV-1, and in particular from the region of said Vpr protein which would correspond to the one ranging from amino acid residues 77 to 92 in the sequence of a reference HIV Vpr protein (for example a Vpr protein of the 89.6 HIV-1 isolate). This peptide, which binds a PP2A holoenzyme or one of its subunits, comprises all or part of the sequence of said Vpr protein which would correspond to the one ranging from amino acid residues 77 to 92 in the sequence of a reference HIV Vpr protein and comprises in particular the region of the Vpr protein which would correspond to the one ranging from amino acid residues 84 to 85 in the sequence of a reference HIV Vpr protein. In particular, the sequence of this peptide comprises amino acid sequence IQ. In particular, in this peptide, the amino acid residues which would correspond to the residues at position 84 and 85 in the sequence of a reference HIV Vpr protein (for example a Vpr protein of the 89.6 HIV-1 isolate) can be respectively I and Q, which is what is found in the Vpr protein of the LAI HIV-1 isolate or in the Vpr protein of the 89.6 isolate of HIV-1²⁵.

Alternatively or cumulatively, the peptide of the invention is a peptide which binds a PP2A holoenzyme (in particular PP2A₁) or one of its subunits, which is selected from the following group of peptides:
a) a peptide comprising or consisting of a peptide sequence chosen from the following group:
   X₇₇HSX₈₀IGIIQQRRX₈₉RNG (SEQ ID No: 3);
   X₇₇HSX₈₀IGIIQQRR (SEQ ID No: 4);
   SX₈₀IGIIQQRRX₈₉R (SEQ ID No: 5);
   IGIIQQRRX₈₉RNG (SEQ ID No: 6);
   X₈₀IGIIQQRRX₈₉ (SEQ ID No: 7);
   X₈₀IGIIQQRR (SEQ ID No: 8);
   IGIIQQRRX₈₉ (SEQ ID No: 9); and
   IGIIQQRR (SEQ ID No: 10);
   wherein X₇₇, X₈₀ and X₈₉ independently designate one or several amino acid residue(s) (preferably one residue), which can be, independently, any amino acid residue(s); and
b) a peptide the sequence of which is distinguished from the sequence envisaged in a) by insertion, substitution or deletion of amino 1 to 5 amino acid residues, in particular 1, 2, 3, 4 or 5 amino acid residue(s).

However, the peptides of the invention do not consist of one of the following sequences:
RHSRIGIIQQRRTRNG (SEQ ID No: 11),
RHSRIGIIQQRR (SEQ ID No: 12),
SRIGIIQQRRTR (SEQ ID No: 13), and
IGIIQQRRTRNG (SEQ ID No: 14).

In a particular embodiment of the invention, a peptide of the invention is selected from the following group of peptides:
a) a peptide comprising or consisting of a peptide sequence chosen from the following group:
   X₇₇HSX₈₀IGIIQQRRX₈₉RNG (SEQ ID No: 3);
   X₇₇HSX₈₀IGIIQQRR (SEQ ID No: 4);
   SX₈₀IGIIQQRRX₈₉R (SEQ ID No: 5);
   IGIIQQRRX₈₉RNG (SEQ ID No: 6);
   X₈₀IGIIQQRRX₈₉; (SEQ ID No: 7);
   X₈₀IGIIQQRR (SEQ ID No: 8);
   IGIIQQRRX₈₉ (SEQ ID No: 9); and
   IGIIQQRR (SEQ ID No: 10);
   or from following group:
   X₇₇HSX₈₀IGIIQQRRX₈₉RNG (SEQ ID No: 3);
   X₇₇HSX₈₀IGIIQQRR (SEQ ID No: 4);
   SX₈₀IGIIQQRRX₈₉R (SEQ ID No: 5);
   IGIIQQRRX₈₉RNG (SEQ ID No: 6);
   X₈₀IGIIQQRRX₈₉ (SEQ ID No: 7);
   X₈₀IGIIQQRR (SEQ ID No: 8); and
   IGIIQQRRX₈₉ (SEQ ID No: 9),
   wherein X₇₇, X₈₀ and X₈₉ independently designate one or several amino acid residue(s), which can be, independently, any amino acid residue(s), and
b) a peptide the sequence of which is distinguished from the sequence envisaged in a) by insertion, substitution or deletion (preferably substitution or deletion and more preferably deletion) of amino acids.

Preferably, the number of amino acids substituted or deleted from a distinct sequence as recited herein (*i.e.,* a sequence which is distinguished from another sequence by insertion, substitution or deletion of amino acids) compared with the original sequence does not exceed 20%, more preferably 10% of the amino acids number constituting the original sequence from which it is derived. Preferably, only amino acids the deletion of which does not affect the *in vitro* binding properties of the peptide to PP2A holoenzyme are substituted or deleted.

In a particular embodiment of the invention, a distinct sequence as recited herein has at least 70% or 80% identity, and preferably at least 90% or 95% identity with the original sequence from which it is derived.

In particular, one distinct sequence is a peptide sequence increasing the binding affinity to a PP2A holoenzyme or one of its subunits compared with the sequence from which it is derived.

A further distinct sequence as defined above is a peptide sequence homologous with an initially identified peptide sequence. The term "homologous peptide" as used in the present invention means a peptide which has a sequence derived from a protein of species other than the initially identified peptide sequence, or a chimeric sequence and for which the primary sequence can be aligned with the peptide sequence initially identified or with a sequence of a reference peptide using a conventional optimum alignment program such as the BESTFIT program (Wisconsin Genetics Software Package, Genetics Computer Group, GCG). In particular, a sequence A will be considered to be homologous with a sequence B if said sequences A and B have at least 50% identity, preferably 75% identity after aligning the sequences using an optimum alignment program such as the BESTFIT program. Preferably again, two sequences are also considered to be homologous if their sequences are quasi-identical, with the exception of a few residues that can represent 10% to 20% variability over the whole sequence. Further, amino acids with the same chemical function (such as Arg and Lys) are considered to be equivalent. The peptides to be analyzed for their binding with a PP2A holoenzyme or one of its subunits are generally selected from fragments of viral, parasitic or cellular proteins, which proteins have been shown to interact *in vivo* or *in vitro* with a PP2A holoenzyme

In a particular embodiment of the invention, in the peptide of the invention,
- X₇₇ and X₈₀ independently designate a single amino acid, which can be chosen from alanine (A) and arginin (R) and/or
- X₈₉ designate a single amino acid, which can be chosen from alanine (A) and threonine (T).

For example, the peptide of the invention can be selected from the following group of peptides:
a) a peptide comprising or consisting of a peptide sequence chosen from the following group:
   AHSAIGIIQQRRTRNG (SEQ ID No: 15);
   RHSAIGIIQQRRTRNG (SEQ ID No: 16);
   AHSRIGIIQQRRTRNG (SEQ ID No: 17);
   AHSAIGIIQQRRARNG (SEQ ID No: 18);
   RHSAIGIIQQRRARNG (SEQ ID No: 19);
   AHSRIGIIQQRRARNG (SEQ ID No: 20)
   RHSRIGIIQQRRARNG (SEQ ID No: 21);
   AHSAIGIIQQRR (SEQ ID No: 22);
   AHSRIGIIQQRR (SEQ ID No: 23);
   RHSAIGIIQQRR (SEQ ID No: 24);
   SAIGIIQQRRAR (SEQ ID No: 25);
   SAIGIIQQRRTR (SEQ ID No: 26);
   SRIGIIQQRRAR (SEQ ID No: 27);
   IGIIQQRRARNG (SEQ ID No: 28);
   AIGIIQQRRA (SEQ ID No: 29);
   AIGIIQQRRT (SEQ ID No: 30);
   RIGIIQQRRA (SEQ ID No: 31);
   AIGIIQQRR (SEQ ID No: 32);
   IGIIQQRRA (SEQ ID No: 33); and
   RIGIIQQRRT (SEQ ID No: 34); and
   RIGIIQQRR (SEQ ID No: 35); and
   IGIIQQRRT (SEQ ID No: 36); and
   IGIIQQRR (SEQ ID No: 10);
   or a peptide comprising or consisting of a peptide sequence chosen from the SEQ ID No: 15 to SEQ ID No: 33; and
b) a peptide, the sequence of which is distinguished from the peptide sequence envisaged in a) by insertion, substitution or deletion (preferably substitution or deletion and more preferably deletion) of amino acids, said sequence nevertheless conserving the properties of binding to a PP2A holoenzyme or one of its subunits.

The invention also concerns a polypeptide, **characterized in that** it is constituted by a repeat or many repeats of a peptide of the invention.

The peptides of the invention are particularly useful in treating certain tumors and in particular human tumors.

A preferred peptide of the invention is **characterized in that** its administration induces or increases apoptosis of target cells or cell lines and in particular tumor cells or tumor cell lines, especially human tumor cells or cell lines and more generally human cells.

In a particular embodiment of the invention, the peptide of the invention competitively inhibits binding of the Vpr native protein from which it derives with a PP2A holoenzyme (in particular PP2A₁) or one of its subunits.

In order to be effective, or more effective, *in vivo* in treating certain tumors, the peptides of the invention can be coupled to a vector that is capable of transferring said peptide into a eukaryotic cell. However, in a preferred embodiment of the invention, as illustrated in the example part of the application, the peptides of the invention themselves are capable of penetrating into cells, meaning that the addition of a vector is not required (but however still possible).

The invention also concerns a compound with a polypeptide framework containing a peptide of the invention as defined above, said compound having a molecular weight in the range 10 to 150 Kdaltons and having the capacity to bind a PP2A holoenzyme or one of its subunits.

Naturally, the invention pertains to means that can be used to synthesize the peptides of the invention. In particular, the invention pertains to a polynucleotide **characterized in that** its sequence consists of the sequence coding for a peptide of the invention.

It may be advantageous to synthesize a polypeptide comprising a repeat of the peptide motifs identified by the process of the invention. As a result, the invention pertains to a polynucleotide **characterized in that** it consists of a multimer of a polynucleotide coding for a peptide of the invention. The invention also pertains to a polypeptide **characterized in that** it is constituted by a repeat of a peptide of the invention.

The invention also pertains to a cell expression vector, **characterized in that** it comprises a polynucleotide of the invention and regulatory sequences allowing expression of a peptide of the invention in a host cell.

The invention also pertains to a method for preparing a peptide as defined herein, comprising transforming a host cell using a cellular expression vector of the invention, followed by culturing the transformed host cell, and recovering the peptide in the culture medium.

The invention further pertains to an antiserum or immunoserum or purified polyclonal antibodies or a monoclonal antibody, **characterized in that** said antibody(ies) or said antiserum or immunoserum is capable of specifically binding a peptide of the invention.

The invention also relates to a method for the preparation of polyclonal or monoclonal antibodies, which is **characterized in that** a peptide of the invention is used as an antigen to elicit antibodies production.

Antibodies specifically directed against the peptides of the invention are obtained, for example, by immunizing an animal after injecting a peptide of the invention, and recovering the antibodies produced. A monoclonal antibody can be obtained using techniques that are known to the skilled person, such as the hybridoma method described by Kohler and Milstein¹⁰.

The antibodies of the invention and the antibodies prepared by the method of the invention are of particular application in immunotherapy. They can act as antagonists for HIV Vpr proteins directed against a PP2A holoenzyme and interfere with the binding of HIV Vpr proteins to a PP2A holoenzyme or to one of its subunits, and thus block HIV development and in particular prevent or inhibit the genesis or progression of AIDS and neuro-AIDS.

Similarly, polynucleotide encoding the peptides of the invention can be directly transferred to the nucleus of target cells, if necessary using suitable vectors, to allow *in vivo* expression of the corresponding peptides, said peptides being susceptible of blocking by competitive inhibition a specific interaction between the PP2A holoenzyme and the Vpr protein from which they derive.

In another aspect, the invention relates to a method of screening one or several peptide(s) derived from the Vpr protein of one or several HIV in particular HIV-1 isolate(s), for its (their) ability to bind a type PP2A holoenzyme or one of its subunits, in particular PP2A₁, and/or its (their) ability to induce or increase apoptosis of tumor cells or tumor cell lines, especially human tumor cells or cell lines, and/or its (their) ability to deliver a compound, in particular a peptide or polypeptide, into a cell, wherein the screened peptides are variants of a Vpr protein, which comprise amino acid sequence IQ. The screened peptides are indeed peptide of the invention, the sequence of which is derived from the C-terminal region of the Vpr protein of a HIV, for example HIV-1, and in particular from the region of said Vpr protein which would correspond to the one ranging from amino acid residues 77 to 92 in the sequence of a reference HIV Vpr protein (for example a Vpr protein of the 89.6 HIV-1 isolate), which peptides comprise amino acid sequence IQ. These peptides are as defined herein.

In a particular embodiment of the invention, the method of screening of the invention comprises:
1) depositing, on a support, the peptides to be analyzed;
2) bringing the support into contact with a solution containing the PP2A holoenzyme or one of its subunits under conditions that allow the peptides present on the support to bind the holoenzyme or one of its subunits; and
3) identifying, on the support, the peptide(s) to which the PP2A holoenzyme or one of its subunits is bound.

In a particular embodiment of the invention, the support is a solid support, for example a cellulose membrane.

Peptide can be deposited in the form of spots on the support, each spot corresponding to the deposit of a peptide with a defined sequence.

In a particular embodiment of the invention, the peptides screened by the method of screening of the invention are selected from the following group of peptides:
a) a peptide comprising or consisting of a peptide sequence chosen from the following group:
   X₇₇HSX₈₀IGIIQQRRXₛ₉RNG (SEQ ID No: 3);
   X₇₇HSX₈₀IGIIQQRR (SEQ ID No: 4);
   SX₈₀IGIIQQRRX₈₉R (SEQ ID No: 5);
   IGIIQQRRX₈₉RNG (SEQ ID No: 6);
   X₈₀IGIIQQRRX₈₉ (SEQ ID No: 7);
   X₈₀IGIIQQRR (SEQ ID No: 8); and
   IGIIQQRRX₈₉ (SEQ ID No: 9);
   wherein X_{77,} X₈₀ and X₈₉ independently designate one or several amino acid residue(s) (preferably one residue), which can be, independently, any amino acid residue(s); and
b) a peptide the sequence of which is distinguished from the sequence envisaged in a) by insertion, substitution or deletion of amino 1 to 5 amino acid residues, in particular 1, 2, 3, 4 or 5 amino acid residue(s),
provided that said peptide does not consist of one of the following sequences:
RHSRIGIIQQRRTRNG (SEQ ID No: 11),
RHSRIGIIQQRR (SEQ ID No: 12),
SRIGIIQQRRTR (SEQ ID No: 13), and
IGIIQQRRTRNG (SEQ ID No: 14).

The invention also pertains to a composition, in particular a pharmaceutical composition, comprising an element selected from a peptide of the invention, a polynucleotide of the invention, a cellular expression vector of the invention, an antibody of the invention or antibodies prepared by the method of the invention. The invention also concerns a composition, in particular a pharmaceutical composition, comprising one or several of these elements, in combination with a pharmaceutically acceptable vehicle.

The invention also relates to an element selected from a peptide of the invention, a polynucleotide of the invention, a cellular expression vector of the invention, an antibody of the invention, antibodies prepared by the method of the invention, a Vpr-derived peptide screened by a method of the invention, or a composition of the invention, for use for delivering one or several compounds in particular one or several polypeptides into a cell.

In a particular embodiment of the invention, the above mentioned elements are **characterized in that** their administration induces apoptosis of tumor cells or tumor cell lines, especially human tumor cells or cell lines. Alternatively or cumulatively, in a particular embodiment of the invention, one or several of the above mentioned elements can advantageously be selected so as to increase apoptosis linked to activation of a cellular PP2A holoenzyme.

Hence, these elements are also for use for inducing or increasing apoptosis of target cells or cell lines and in particular tumor cells or tumor cell lines, especially human tumor cells or cell lines and more generally human cells. Of course, the invention also concerns the use of at least one of these elements in preparing a drug that can induce or increase apoptosis of target cells or cell lines and in particular tumor cells or tumor cell lines, especially human tumor cells or cell lines and more generally human cells. These elements can be used for the treatment of disorders induced by or associated with a defective regulation of cell death which results in an abnormal decrease of cell death, or of any specific pathology in which cell death may be at least a part of the therapy. They can be administered to a human or non human host.

HIV infection may result in specific expression of proteins comprising sequences of peptides of the invention. The sequences encoding the peptides of the invention can thus be used as a probe to detect, in a specific manner from RNA extracted from a biological sample from a patient, a specific HIV-infection.

Similarly, antibodies of the invention or antibodies prepared by the method of the invention can be used to specifically recognize, in a biological sample from a patient, peptide sequences contained in Vpr proteins expressed during HIV-infection.

Thus, the invention concerns the use of a polynucleotide of the invention or an antibody of the invention or antibodies prepared by the method of the invention, in the *in vitro* diagnosis of HIV infection.

Another aspect of the invention relates to a kit comprising or consisting of:
- one or several set(s) of primers chosen for their ability to allow amplification of the nucleic acid sequence encoding a region in a Vpr protein which comprises the X₈₄X₈₅ domain; and/or
- one or several set(s) of probes enabling the detection of the amplified products.

Said kit is appropriate for analyzing, in particular *in vitro* or *ex vivo,* the Vpr protein of a HIV, in particular a HIV-1, as disclosed herein.

In another aspect, the invention further relates to a method of screening a test compound for its ability to interfere with (in particular to competitively inhibit) the binding of a HIV Vpr protein or of one or several peptide(s) or polypeptide(s) derived from a HIV Vpr protein, to a PP2A holoenzyme (in particular PP2A₁) or to one of its subunits, which peptides or polypeptides comprise the amino acid residues of the Vpr protein which would respectively correspond to the residues at position 84 and 85 in the sequence of a reference HIV Vpr protein. Said method comprising or consisting of the steps of:
a) providing an assay system comprising:
   - a Vpr protein of a HIV, in particular a Vpr protein of a HIV-1, or a fragment of said Vpr protein, said fragment comprising the amino acid residues of said Vpr protein which would respectively correspond to the residues at position 84 and 85 in the sequence of a reference HIV Vpr protein, and said fragment retaining the ability of said Vpr protein to bind a PP2A holoenzyme or one of its subunits; and
b) contacting said assay system with the test compound in conditions enabling the interaction between the test compound and the PP2A holoenzyme or one of its subunits;
c) determining whether said test compound interferes with the binding of the Vpr protein or a fragment thereof to the PP2A holoenzyme or to one of its subunits.

In a particular embodiment of the invention, in the Vpr protein or the fragment thereof provided in step a), X₈₄ is I and X₈₅ is Q. For example said Vpr protein can be a Vpr protein of the LAI isolate of HIV-1 or of the 89.6 isolate of HIV-1 ²⁵.

The fragment of the Vpr protein provided in step a) can also be obtained by the method disclosed herein for screening Vpr-derived peptide(s) for their ability to bind a PP2A holoenzyme or one of its subunits.

As used herein, the term "compound" refers to inorganic or organic chemical or biological compounds either natural (isolated) or synthetic, and especially encompass nucleic acids, proteins, polypeptides, peptides, glycopeptides, lipids, lipoproteins and carbohydrates. Said compound can be particular a peptide, a polypeptide, a chemical drug or an antibody.

An example of peptide or polypeptide derived from a HIV Vpr which can be used in the screening method of the invention is a peptide or polypeptide derived from the region of a Vpr protein of a HIV (in particular a HIV-1) which would correspond to the region ranging from amino acid residues 77 to 92 in the sequence of a reference HIV Vpr protein, and more generally, a peptide or polypeptide derived from the C-terminal region of a Vpr protein of a HIV (in particular a HIV-1). In a particular embodiment of the invention, the Vpr-derived peptides or polypeptides which are used in the screening method of the invention comprise amino acid sequence IQ and/or comprise amino acid residue X₈₄ and/or amino acid residue X₈₅ (as defined herein) of the Vpr protein from which they are derived. In a more particular embodiment, amino acid sequence X₈₄X₈₅ is IQ in the Vpr-derived peptides or polypeptides which are used for screening in the method of the invention.
The invention also relates to the compounds, peptides or polypeptides identified by a method of screening of the invention.

### BRIEF DESCRIPTION OF THE FIGURES

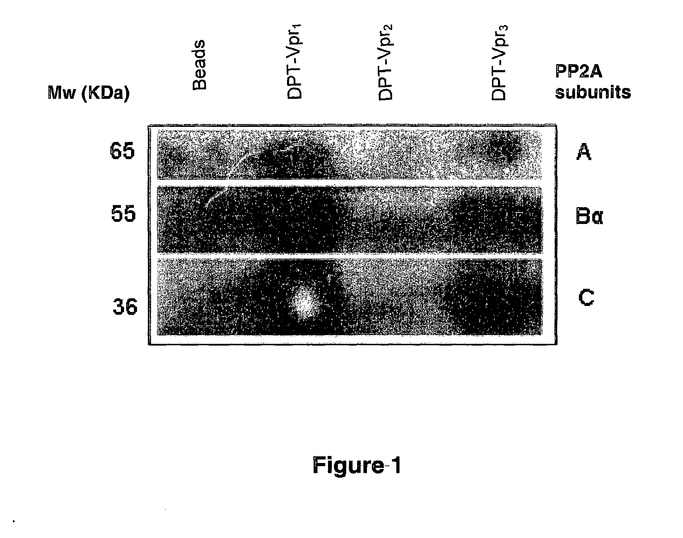

**Figure 1****: Co-precipitation of DPT-Vpr peptides with PP2A₁ in HeLa cell extracts.** Control magnetic beads coupled with streptavidin alone or conjugated with DPT-Vpr peptides, were incubated with cells extracts (5.10⁵ cells) at a concentration of 100 µM. Identification of bound proteins in pull-down experiments was realized by immunoblotting using antibodies against PP2A₁.

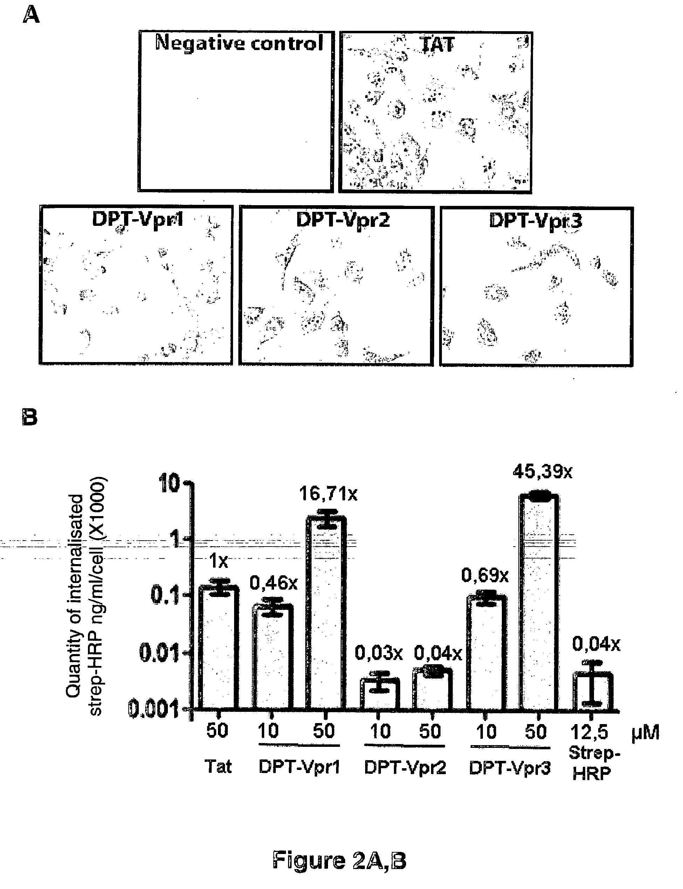

**Figure 2****: Effect of DPT-Vpr peptides on HeLa cell penetration and intracellular delivery of streptavidin-peroxydase. (A)** For penetration and localization analysis, cells were incubated with 150 µM of DPT-Vpr peptides for 2 hrs at 37° C and before microscopy analysis, presence of DPT-Vpr peptides is revealed by a colorimetric test DAB. **(B)** Intracellular delivery of streptavidin-peroxydase by biotinylated-DPT-Vpr and TAT peptides in HeLa cells. Streptavidin-peroxydase coupled with biotinylated peptides were incubated for 6 hrs at 37°C and internalized complexes were visualized by a colorimetric test OPD as indicated herein in part "A.2. Methods".

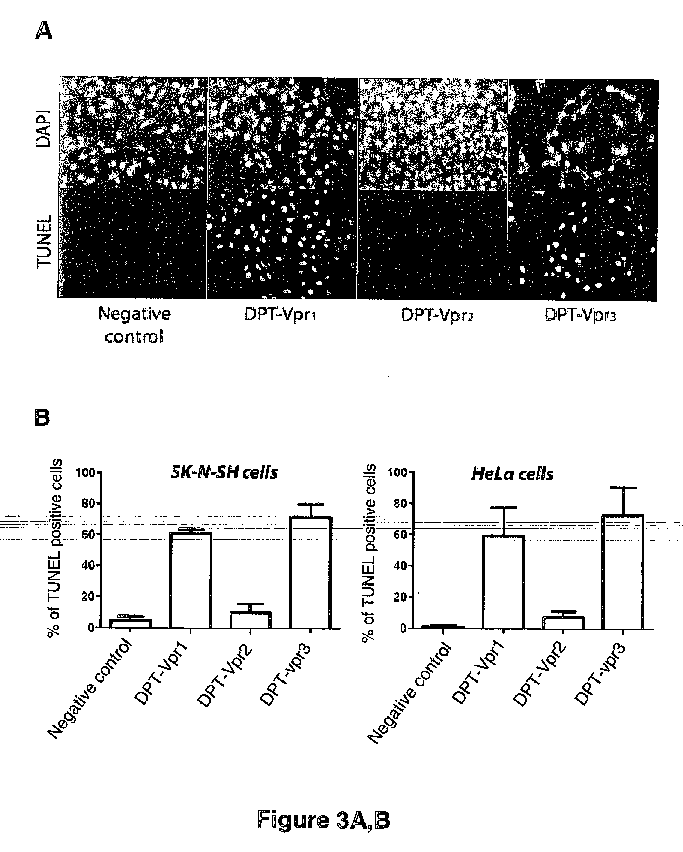

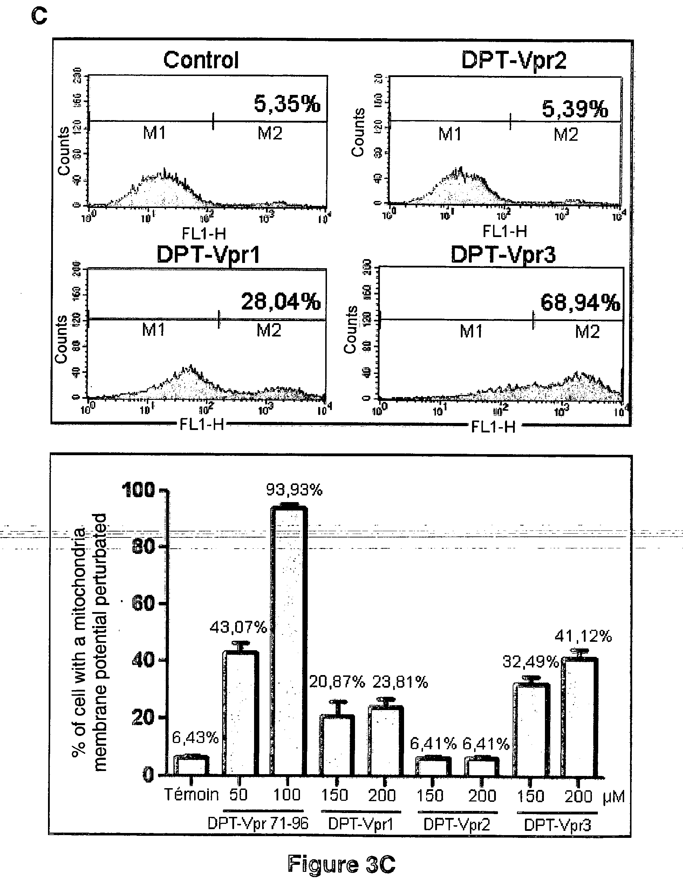

**Figure 3****:** Effect of DPT-Vpr peptides on cell death In A nuclear morphology illustrated with SK-N-SH cells was evaluated with DAPI (blue) and DNA strand breaks were identified using TUNEL labeling (green). In B histograms show percentages of positive SK-N-SH cells (left panel) or HeLa cells (right panel) detected in TUNEL results.(C) To monitor apoptosis in Jurkat cells treated with DPT-Vpr peptides, we used Annexin V (upper panel) or DiOC₆ (lower panel) assays as described in Methods. Control Peptide Vpr₇₁₋₉₆ comprises both the Vpr₇₁₋₈₂ mitochondrial death domain and the Vpr₇₇₋₉₂ domain. For DiOC₆ statistical analysis was by Anova and significance was set at P<0.05.

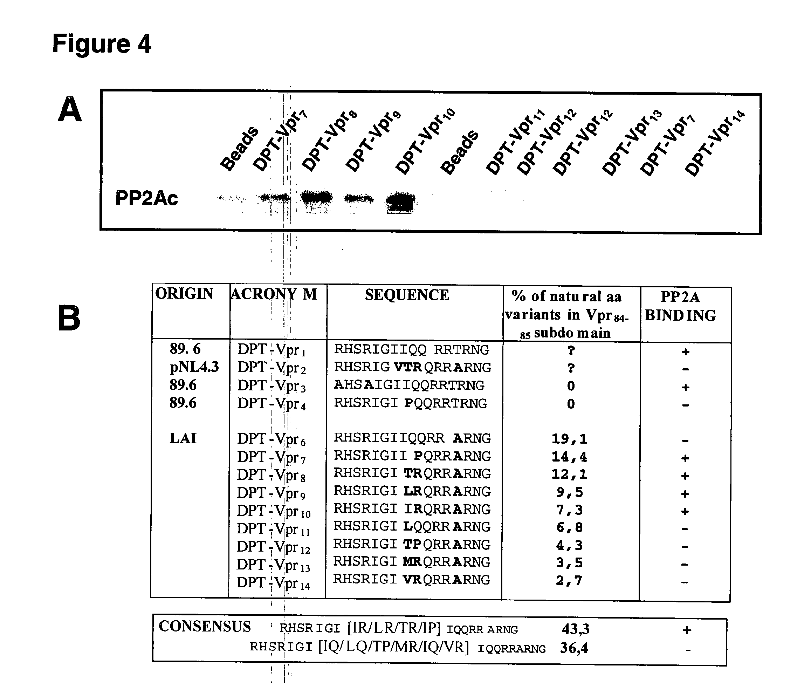

**Figure 4****:** Effect of DPT-Vpr peptides containing sequences with A79/T substitution or variable Vpr₈₄₋₈₅ combination on PP2A₁ binding in SK-N-SH cells. (A) Co-precipitation of DPT-Vpr peptides containing variable Vpr₈₄₋₈₅ combination and A79/T substitution with PP2A₁ in SK-N-SH cell extracts. (B) Deduced consensus motif for PP2A₁ binding.
**Figure 5****: Principle of the Multiplex Ligation Probe Assay (MLPA).**

### EXAMPLES: The HIV-1 89.6 Vpr₇₇₋₉₂ sequence is a new isolate-dependent death domain involved in AIDS and neuro-AIDS genesis

Vpr, an accessory protein of human immunodeficiency virus type 1 (HIV-1) can cause cell cycle arrest and cell death in several cell types¹⁻³. Based on *in vitro* binding assays, we have previously established that the Vpr₇₇₋₉₂ domain containing the sequence RHSRIGIIQQRRTRNG (SEQ ID No: 11) deduced from the residues 77-92 in the C terminal portion of the HIV-1 89.6 Vpr protein is a new PP2A binding domain⁴.

Experiments combining pull-down, cell penetration and cell death analyses in HeLa, Jurkat and SK-NSH cells indicate that the PP2A₁ binding sequence from Vpr₇₇₋₉₂ is also a new cell penetrating death domain. Analysis of the isolate HIV-1 sequences retrieved from the Los Alamos data base shows that the Vpr₇₇₋₉₂ domain contains a highly variable micro domain including two highly variable residues located in position 84 and 85. In addition, I84P mutation in Vpr 89.6 or IIQ/VTR₈₃₋₈₅ substitution from proviral pNL4.3 indicate that isolate-dependent variability within Vpr₈₄₋₈₅controls the PP2A₁ binding and the death properties of the Vpr₇₇₋₉₂domain.

The results presented here suggest that the 89.6 Vpr₇₇₋₉₂ domain properties, PP2A₁ binding, cell penetrating and cell death induction represent a new element participating to human AIDS pathology. In addition we have identified by the analysis of Vpr sequences from different HIV-1 isolates, a highly variable micro domain controlling the activities of Vpr₇₇₋₉₂ domain. The sequence analysis of this domain will lead to develop a new diagnostic approach to predict the potential evolution of HIV Associated Dementia (HAD) and long-term non progression.

We have previously described⁴ the sequence corresponding to the Vpr₇₇₋₉₂ domain of HIV-1 89.6 isolates as a PP2A₁ binding domain. We have here characterized the Vpr₇₇₋₉₂ domain as a cell penetrating death domain. Furthermore in contrast to the non penetrating but highly conserved mitochondriotoxic domain (Vpr₇₁₋₈₂: HFRIGCRHSRIG; SEQ ID No: 37) our data show that Vpr₇₇₋₉₂ is also an isolate-dependent death sequence which is inactivated in proviral DNA pNL4.3 sequence.

### A. Material and methods

### A.1. Material

**Cell Culture and Reagents.** Adherent HeLa cells (American Type Culture Collection, Manassas, VA) were cultured in Dulbecco's modified Eagle's medium (D-MEM Invitrogen, Carlsbad, CA) as exponentially growing sub confluent monolayer on micro plates or in 12-, 24- or 96- well plates. Jurkat lymphoid T cells (clone E6; Americaine Type Culture Collection) were cultured in RPMI 1640 Glutamax Medium (Gibco; Invitrogen). Both cell lines were cultured in medium supplemented with 10% fetal calf serum (Invitrogen). Adherent SK-N-SH cells (neuroblastoma cells (ATCC HTB11)) were cultured in D-MEM Glutamax-I-High Glucose (Gibco) with 10% fetal calf serum (Gibco/BRL).

**Peptides.** High-performance liquid chromatography-purified NH₂-biotinylated peptides (purchased from Neosystem) were synthesized by solid-phase peptide synthesis. Peptides were dissolved in DMSO and stored at 4°C until use, or in half of EtOH and 150mM NaCl and stored at -20°C until use.

**Antibodies.** Characterization of PP2A antibodies (polyclonal guinea pig anti-A, or anti-B and anti-C subunits) has been described previously¹¹. Anti-Guinea Pig-HRP used to 1/5000 (DAKO PO141).

**Kits and Reagents.** Lab-Tek II Chamber Slide w/Cover, 8 well (Ref 154534, Nalge Nunc International), streptavidin Horse Radish Peroxydase conjugate (Euromedex), 3,3'-diaminobenzydine tetrahydrochloride (DAB) of DAB Substrate Kit for Peroxidase (Vector laboratories), "complete mini EDTA free" protease inhibitor cocktail from Roche, O-Phenylenediamine dihydrochloride (OPD) tablet from Sigma chemical, Annexin-V-FITC from Roche, DiOC₆(3) from Sigma, Kit DeadEnd^{™} Fluorometric TUNEL system product (Promega), FIuorSave^{™} Reagent from Calbiochem, Vectashield mounting medium for fluorescence, with DAPI (Vector Laboratories).

### A.2. Methods

**A.2.1. Pull down assays to detect interaction of biotinylated peptides with PP2A₁ target.** Biotinylated peptides were preincubated 2 h at 100µM or 200µM (in final concentration with lysate) at room temperature with 30µl of streptavidincoated immunomagnetic beads (Calbiochem, San Diego CA). During this time, 1×10⁶ HeLa cells at 90% of confluence per peptide tested were washed with PBS, trypsined and harvested with 1 ml of PBS, centrifugated at 600g 4°C 10 min and lysed 10 min on ice_with 400µl of lysis buffer (50mM Tris-HCl pH 7.4, 200mM NaCl, 10M EDTA, 20% Glycerol, 1% Nonidet P-40, 1mM phenylmethylsulfonyl fluoride (PMSF), 10mM NaF, 1 mM orthovanadate, and "complete mini EDTA free" protease inhibitor cocktail from Roche). Lysates were clarified at 13,000g for 10 min at 4°C (for SK-N-SH cells a pre-clearing of 1 hour in rotation at 4°C with 50µl of beads for 800µl of lysates clarified, the magnetic beads are puledl down) and after rotation with supernatant at 4°C for 2 h, biotinylated peptides were pulled down with streptavidin magnetic beads and washed twice with 700µl of lysis buffer. Bound proteins and clarified lysates were analyzed by western blotting using PP2A₁ antibodies.

**A.2.2. Cellular Localization of Biotinylated Peptides.** A total of 4×10⁴ exponentially proliferating HeLa cells growing subconfluent monolayer were seeded per well on Lab-Tek II Chamber Slide w/Cover, 8 well (Ref 154534, Nalge Nunc International) and incubated at 37°C, 5% CO₂. 48 h later, biotinylated peptides were added to the cells at different concentrations and for different periods of time at 37°C, 5% CO₂. Cells were washed twice in phosphate-buffered saline (PBS), and fixed with absolute ethanol for 10 min at -20°C before adding 100µM of streptavidin HRP conjugate (Cat # 18-152, Euromedex). After a 30 min-incubation at 37°C, cells were rinsed twice in PBS and incubated with a solution of DAB is used as described by the manufacturer (Vector laboratories; reference SK-4100) for 10 min around. Cells were rinsed in distilled water and mounted for analyzed by microscopy.

**A.2.3. Quantification of Peptide Internalization.** Before incubation, peptides were pre-incubated 1 h with streptavidin HRP conjugate in a 4/1 ratio. HeLa cells at 80% of confluence were incubated with different concentrations of peptide in 24-well plates. After 6 h or 24 h, cells were washed twice in PBS, trypsined (Trypsin EDTA, Invitrogen) and harvested in 1ml with PBS and counted. Cells were lysed in 300µl of 0.1 M Tris pH 7.4, and 0.5% Nonidet P-40 buffer for 10 min on ice. A total of 50µl of cell lysate was mixed with 50µl of OPD buffer (51.4 mM Na2HPO4 and 24.3 mM citric acid) then mixed with 100µl of OPD solution (one OPD tablet from Sigma in 100ml of OPD buffer in which 40 µl of 30% hydrogen peroxide was added just before use). After 10 to 20 min, reaction was stopped by addition of 100µl of 1 M HCl, and optical density was read at 490 nm.

**A.2.4. Annexin-V assays and evaluation of mitochondrial membrane potential** (Δψm). For detection of apoptotic cells, we used Jurkat cells (2.4.10⁵ cells in 400µl of complete RPMI-1640 medium) incubated or not with okadaic acid 1 h at 37°C and 5% CO₂ and with different concentrations of peptide. After 3 h, cells were washed in PBS and we used an Annexin-V-FITC (Roche) for the assessment of outer leaflet exposure of phosphatidylserine (PS) in the plasma membrane to detect early the apoptotic cells. Staining was performed according to the manufacturer's instructions. Or we used the cationic Dye DiOC₆(3), which localizes to intact mitochondria to measure the Δψm. The DiOC₆(3) was added at 20nM during 15 at 30 min at 37°C in the dark. Cells were washed in PBS and immediately analyzed by flow cytometry. For the twice assays a total of 20,000 cells were analyzed by flow cytometry in a FACScalibur flow cytometer (BD Biosciences, San Jose, CA).

**A.2.5. Apoptosis was monitored by *in situ* detection of fragmented DNA (TUNEL).** DNA fragmentation and terminal deoxynucleotidyl transferase (Tdt)-mediated dUTP nick end-labeling (TUNEL) assay DNA fragmentation occurs at a later stage in apoptosis and this assay utilizes the generated 30 free hydroxyl groups at the ends of the fragmented DNA, to be labeled with fluorescein dUTP in the presence of TdT. TUNEL assays were performed using the Kit DeadEnd^{™} Fluorometric TUNEL system product (G3250 Promega). A total of 4×10⁴ exponentially proliferating SK-N-SH or HeLa cells growing sub confluent monolayer were seeded per well on Lab-Tek II Chamber Slide w/Cover, 8 well (Ref 154534, Nalge Nunc International) and incubated at 37°C, 5% CO₂. 18 h later, biotinylated peptides were added to the cells at different concentrations and for different periods of time at 37°C, 5% CO₂. After incubation, cells were rinsed twice with 1X PBS samples and were fixed with 4% paraformaldehyde solution in 1X PBS, for 25 min at 4°C. Slides were rinsed twice with 1X PBS during 5 min and permeabilizated with a solution consisting of 0.2% Triton X-100 in 1X PBS for 5 min at Room Temperature (RT). Cells were washed twice with 1X PBS prior to labeling. Cells are equilibrated with "Equilibration Buffer" purchased by the kit 10 min at RT. TUNEL reaction labeling was performed by using the specified amounts of the "Equilibration Buffer", "Nucleotide Mix" and "rTdT Enzyme" and incubated for 60 min in the dark, at 37°C in a humidified chamber. To stop the reaction the slides is immerged 15 min in SSC 2X (in 1X PBS) purchased by Promega. Slides are rinsed three times in 1X PBS and mounted with Vectashield with DAPI. Results were analyzed by fluorescence microscopy.

### B. Results

### B.1. The Vpr₇₇₋₉₂ sequence from HIV-1 89.6 isolate but not from proviral pNL4.3 interacts with PP2A₁ in cell extracts

We have previously described the sequence corresponding to the Vpr₇₇₋₉₂ domain of HIV-1 89.6 isolate as a PP2A₁ binding domain. The two homologous HIV-1 Vpr proteins from LAI isolate or from proviral DNA pNL4.3, that display 96% of sequence identity, have been commonly and indistinctly used in numerous studies.

However, as illustrated in Table 1, we found that the three residues corresponding to Vpr₈₃₋₈₅ sequence from 89.6 or pNL4.3 sequences are different suggesting a possible difference in their ability to interact with PP2A₁. To test this hypothesis, we chemically synthesized biotinylated peptides corresponding to the residues 77-92 in the C terminal portion of both Vpr 89.6 and Vpr_{pNL4.3} (see Table 2 for sequence details).

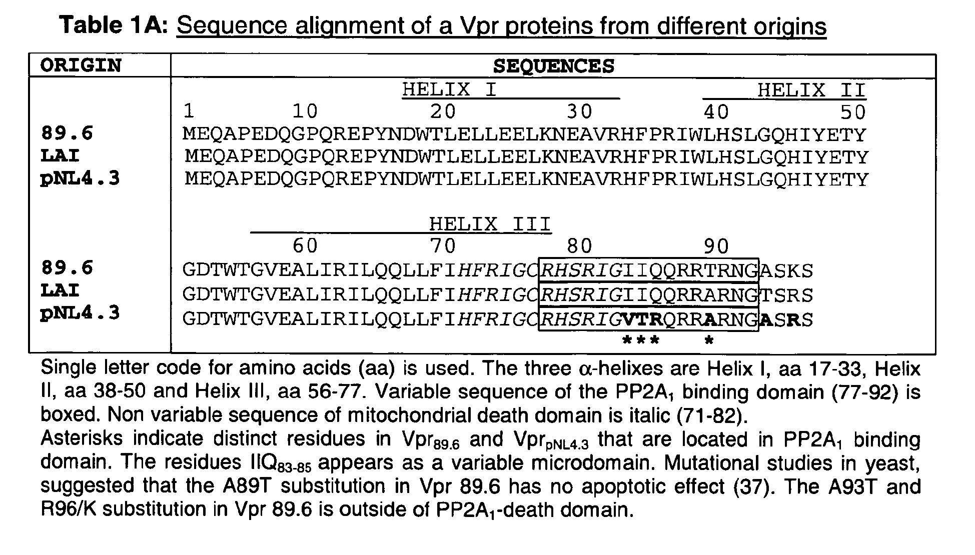

**Table 2: Origin and sequences of peptides containing Vpr sequences used in this study -A single letter amino acid code is used for all peptides.**

| **ORIGIN** | **ACRONYM** | **SEQUENCES** | **PP2A₁ binding** | **Cell Internalisation** | **Cell Death** |
|---|---|---|---|---|---|
| **89.6** | **DPT-Vpr**₁ | RHSRIGIIQQRRTRNG | + | + | + |
| **pNL4.3** | **DPT-Vpr**₂ | RHSRIG**VTR**QRR**A**RNG | - | - | - |
| **89.6** | **DPT-Vpr**₃ | **A**HS**A**IGIIQQRRTRNG | + | + | + |
| **89.6** | **DPT-Vpr**₄ | RHSRIGI**P**QQRRTRNG | -** | -** | -** |
| **pNL4.3** | ***Vpr**₇₁₋₉₆ | HFRIGCRHSRIG**VTR**QRR**A**RNGASRS | ? | ? | + |

As shown in figure 1 pull down assays indicate that the two peptides, DPT-Vpr₁, corresponding to wild Vpr₇₇₋₉₂ sequence from HIV-1 89.6, and DPT-Vpr₃, a variant containing the two R77A and R80A mutations of the Vpr 89.6 sequence, can co-precipitate PP2A₁ from HeLa cell extracts. In contrast PP2A₁ is not detected in pull down experiments with DPT-Vpr₂ corresponding to a wild type Vpr₇₇₋₉₂ sequence from HIV-1 pNL4.3. These data suggest that the IIQ/VTR substitution in the Vpr₇₇₋₉₂ sequence from HIV-1 pNL4.3 prevents PP2A₁ binding.

### B.2. Vpr₇₇₋₉₂ domain of HIV-1 89.6 is a new cell penetrating sequence

It is well established that TAT peptides derived from the cell penetrating protein HIV-1 TAT allow cell penetration and intracellular delivery of proteins. TAT transduction depends on various factors, and current protocols permit the transduction into many cells¹⁴. Since Vpr is a known penetrating protein we hypothesized that DPT-Vpr peptides containing the Vpr₇₇₋₉₂ sequence could also display transduction activity. To test this hypothesis biotinylated DPT-Vpr peptides were used to investigate their ability to penetrate and to deliver a protein marker (Streptavidin-HRP) into lymphoid Jurkat or adherent HeLa cells. As shown in figure 2A, DPT-Vpr₁, DPT-Vpr₂ and DPT-Vpr₃ peptides penetrate into HeLa cells. However Tat and DPT-Vpr peptides show significant differences in their intracellular localization. While DPT-Vpr₂ display cytoplasmic and nuclear/nucleolar localization similar to TAT, DPT-Vpr₁ and DPT-Vpr₃ only localized in the cytoplasm although DPT-Vpr₃ also shows a discrete perinuclear staining. Furthermore as shown in figure 2B, we quantified in HeLa cells the cargo effect of biotinylated DPT-Vpr peptides in comparison to a biotinylated TAT-derived penetrating peptide. Surprisingly we found that DPT-Vpr₁ and DPT-Vpr₃ peptides are respectively 10.6 and 28.7 fold more efficient to internalize streptavidin-HRP conjugated molecules than TAT-peptide. In contrast, no internalization effect is detected for DPT-Vpr₂.

### B.3. The PP2A-interacting sequence of HIV-1 Vpr 89.6 is a new death domain

We used TUNEL assay to monitor apoptosis in adherent HeLa or SK-N-SH cells. As shown in figure 3A both DPT-Vpr₁ and DPT-Vpr₃ provoked a strong apoptosis in both Hela and SK-N-SH cells. In contrast, DPT-Vpr₂ has no significative effect. Furthermore experiments using Annexin V (Fig.3C upper panel) indicated that DPT-Vpr₁ and DPT-Vpr₃ but not DPT-Vpr₂, induced apoptosis in non-adherent Jurkat cells. In addition, we found that similarly to Vpr₇₁₋₉₆, a peptide containing the mitochondrial death domain from pNL4.3 (12), DPT-Vpr₁ and DPT-Vpr₃ but not DPT-Vpr₂ induced a decrease in mitochondrial membrane potential (Δψm) (Fig.3C lower panel). Together these results indicate that the Vpr₇₇₋₉₂ sequence is a new PP2A₁-binding death domain.

### B.4. Bioinformatics analysis of Vpr₇₇₋₉₂ sequences among different HIV-1 isolates

The conservation of Vpr₇₇₋₉₂ domain between different HIV-1 isolates was analyzed by retrieving 3600 Vpr sequences from Los Alamos library. These sequences were aligned using clustal¹⁵ and the frequency of each amino acid in Vpr₇₇₋₉₂ domain determined. A consensus sequence of Vpr₇₇₋₉₂ domain derived from the 3600 sequences analyzed is also presented (Table 3). The different residue positions and amino acid residues appeared highly conserved (>95%), contrasting with a hypervariable microdomain restricted to residues 84 and 85 which are frequently occupied by three different amino acids (I/L/T and Q/T/R respectively in more than 80% of the analyzed sequences). The sequence variants DPT-Vpr₃ and DPT-Vpr₄ corresponding to double R77A and R80A or single I84P mutation respectively are not found in natural Vpr sequences analyzed. In contrast I/V₈₃mutation in residue 83 from proviral pNL4.3 was found at very low frequency. We used nine distinct biotinylated DPT-Vpr₁ mutant sequences containing A79/T substitution and recovering 80% of IQ₈₄₋₈₅ frequency to correlate the variability of Vpr₇₇₋₉₂ domain with its biological properties. As shown in Fig. 4A pull down experiments with SK-N-SH cell extracts indicate that peptides containing (IR/LR/TR/IP) residues co-precipitate PP2A₁ subunits. Together these studies establish a consensus motif based on PP2A₁-binding properties of Vpr₇₇₋₉₂ domain (Fig.4B).

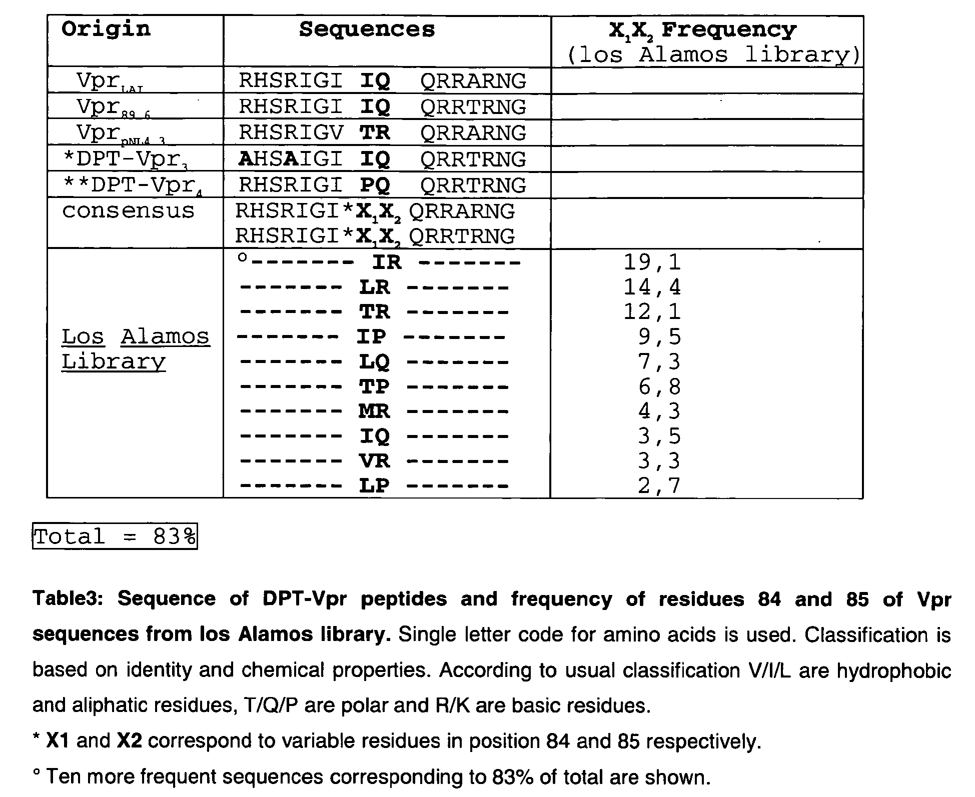

### C-Discussion

### The Vpr₇₇₋₉₂ PP2A₁ binding sequence is a new HIV-1 cell penetrating and cell death domain

This study builds upon our previous biochemical identification of the PP2A₁ binding sequence, the Vpr₇₇₋₉₂ domain, located in the c-terminal portion of HIV-1 Vpr from 89.6 isolate⁴.

Here, we found that the Vpr₇₇₋₉₂ domain is a new cell penetrating and cell death domain. In accordance with the DPT concept we called this new DPT-peptide, DPT-Vpr₁.

In contrast, the mitochondriotoxic domain Vpr₅₆₋₇₇, which is also a cell death domain, does not allow cell penetration and intracellular delivery of polypeptides.

It has been reported that, similarly to cationic peptides, the Tat peptide derived from the HIV-1 TAT protein possessed protein transduction activity. Interestingly when compared to the activity of Tat, DPT-Vpr₁ has more potent activity in transporting streptavidin HRP molecules. Surprisingly the DPT-Vpr₂ peptide sequence, corresponding to the Vpr₇₇₋₉₂ domain from proviral pNL4.3 Vpr DNA that contains the IIQ/VTR₈₃₋₈₅ substitution, has lost PP2A₁ binding (figure 1), intracellular transduction capacity (figure 2) and cell death activity (figure 3). Further studies in Vpr 89.6 isolate indicate that PP2A₁ binding, cell penetrating and cell death properties remain unaffected by R/A substitution of the two R77 and R80 residues in Vpr₇₇₋₉₂ domain of DPT-Vpr₃ peptide. In addition, bioinformatics analysis indicates that Vpr₇₇₋₉₂ domain contains a highly variable micro domain defined by Vpr₈₄₋₈₅ residues. Furthermore, consistent with a functional role of isolate variability within this micro domain, I84P mutation in Vpr 89.6 or IIQ/VTR₈₃₋₈₅ substitution from proviral pNL4.3 Vpr₇₇₋₉₂ sequences suppress PP2A₁ binding and cell death. These results imply a role for Vpr₇₇₋₉₂ in AIDS progression.

### HIV-1 isolate-dependent Vpr₇₇₋₉₂ domain: a new tool to study long-term progression and neuroAIDS

In contrast to the highly conserved mitochondrial Vpr₇₁₋₈₀ death domain, the isolate-variability found within PP2A₁-dependent Vpr₇₇₋₉₂ death domain could play a role in HIV-1 physiopathology. Taking in account that Vpr is also present in the body fluids of HIV-1 patients including plasma and cephalo rachidian liquid (CRL), otherwise the CD4 HIV-1 infected cells, soluble Vpr proteins or C-terminal processed fragments derived from various isolate with high PP2A₁ binding affinity could also induce a bystander death effect in non-infected CD4 cells.

Cell death experiments in SK-N-SH cells suggest that Vpr₇₇₋₉₂ LAI sequence is also potently active in neuronal cells. Interestingly, neurocytopathic effects induced by recombinant Vpr molecule in neuronal cells, suggest that HIV-1 Vpr is involved in the genesis of neurological disorders¹⁶⁻²⁰. Furthermore, in contrast to peptides containing the mitochondrial toxic sequence (residues 71-82), the peptide sequences spanning the C-terminal domain of Vpr (residues 52-96 or 70-96) induced apoptosis in cultured rat cortical and striatal neurons²¹. Together these results suggest that a direct interaction between Vpr and ANT alone is inefficient to induce apoptotic pathway in neurons¹⁹⁻²⁰. In this context, recent data indicate that soluble forms of Vpr are present in the extracellular medium of HIV-1-producing cells. In addition, a specific proteolytic processing of HIV-1Vpr occurring at a very well conserved protein convertase cleavage site, ₈₅RQRR₈₈, located within C-terminal domain of HIV-1 Vpr pNL4.3 was described²³. It is noteworthy that such cleavage occurs in variable residue of Vpr₇₇₋₉₂ suggesting that isolate containing PP2A₁-interacting sequences are not subjected to his particulate processing.

Together, these results suggest that sequence variability in Vpr₇₇₋₉₂ sequences leading to inactivation of the new PP2A₁-dependant Vpr₇₇₋₉₂ death domain could favor long-term non progression. In contrast functionally active PP2A₁ binding Vpr₇₇₋₉₂ death sequences in HIV-1 isolates is a positive event for AIDS progression and potential development of HAD. Particularly in the case of HAD, since HAART treatments don't cross blood-brain barrier, it will be important to identify new DPT (Drug Phosphate Technology)-peptides or/and to design new chemical drugs that compete with Vpr₇₇₋₉₂ PP2A₁ binding sequence. For example identification of the residues of PP2A-A subunit interacting with the Vpr₇₇₋₉₂ sequence from 89.6 isolate, will allow the design and the synthesis of new DPT-competitor peptides.

### REFERENCES

1. Alimonti JB, Ball TB, Fowke KR: Mechanisms of CD4+ T lymphocyte cell death in human immunodeficiency virus infection and AIDS. J Gen Virol 2003, 84:1649-1661
2. Le Rouzic E, Benichou S. The Vpr protein from HIV-1: distinct roles along the viral life cycle.Retrovirology. 2005 22:11.
3. Stewart SA, Poon B, Jowett JB, Chen IS: Human immunodeficiency virus type 1 Vpr induces apoptosis following cell cycle arrest. J Virol 1997, 71:5579-5592.
4. Patent applications FR 01/10139 and WO 03/011898.
5. Jacotot, E, et al. The HIV-1 viral protein R induces apoptosis via a direct effect on the mitochondrial permeability transition pore. J. Exp. Med. 2000. 191:33-46.
6. Jacotot E, Ferri KF, Ej Hamel C, Brenner C, Druillenec S, Hoebeke J, Rustin P, Metivier D, Lenoir C, Geuskens M, Vieira HLA, Loeffler M, Belzacq A-S, Briand J-P, Zamzami N, Edelman L, Xie ZH, Reed JC, Roques BP, Kroemer G. Control of mitochondrial membrane permeabilization by adenine nucleotide translocator intercating with HIV-1 viral protein R and Bcl-2. J. Exp. Med. 2001.193:509-520.
7. A. Garcia, X. Cayla, J. Guergnon, F, Dessauge, V, Hospital, MP, Rebollo, A, Fleischer, A. Rebollo. Serine/threonine protein phosphatases PP1 and PP2A are key players in apoptosis. Biochimie. (2003) 85 :721-726.
8. Virshup DM, Shenolikar S. From promiscuity to precision: protein phosphatases get a makeover. Mol Cell. 2009 13:537-545.
9. J. Guergnon, F. Dessauge, V. Dominguez, J. Viallet, X. Cayla, A. Rebollo, V.Yuste, S.Susin, PE. Bost and A. Garcia Use of penetrating peptides interacting with PP1/PP2A proteins as a basis for a new Drug Phosphatase Technology. Mol. Pharmacol. (2006) 69 :1115-1124.
10. Kohler, G.; Milstein, C. (1975). Continuous cultures of fused cells secreting antibody of predefined specificity. Nature 256 (5517): 495-497.
11. Bosch et al. Bosch M, Cayla X, Van Hoof C, Hemmings B, Ozon R, Merlevede W, and Goris J (1995) The PR55 and PR65 subunits of protein phosphatase 2A from Xenopus laevis: molecular cloning and developmental regulation of expression Eur. J. Biochem. 230: 1037-1045.
12. Arunagiri C., Macreadie I., Hewish D., Azad A. A. A C-terminal domain of HIV-1 accesssory protein Vpr is involved in penetration, mitochondrial dysfunction, and apoptosis of human CD4+ lymphocytes. Apoptosis 1997 2 : 69-76.
13. Schwarze SR, Ho A, Vocero-Akbani A, and Dowdy S.F. (1999) In vivo protein transduction: delivery of a biologically active protein into the mouse. Science 285 :1569-1572.
14. Thompson JD, Gibson, TJ, Plewniak F, Jeanmougin F, Higgins DG (1997) The CLUSTAL_X windows interface: flexible strategies for multiple sequence alignment aided by quality analysis tools Nucleic Acids Res 25: 4876-4882.
15. Deniaud A., Brenner C., Kroemer G. Mitochondrial membrane permeabilization by HIV-1 Vpr. Mitochondrion 2004 4 : 223-233
16. Patel CA, Mukhtar M, Harley S, Kulkosky J, Pomerantz RJ: Lentiviral expression of HIV-1 Vpr induces apoptosis in human neurons. J Neurovirol 2002, 8:86-99.
17. Piller SC, Ewart GD, Jans DA, Gage PW, Cox GB: The amino-terminal region of Vpr from human immunodeficiency virus type 1 forms ion channels and kills neurons. J Virol 1999,73:4230-4238.
18. Huang MB, Weeks O, Zhao LJ, Saltarelli M, Bond VC: Effects of extracellular human immunodeficiency virus type 1 vpr protein in primary rat cortical cell cultures. J Neurovirol 2000, 6:202-220.
19. Sabbah EN, Roques BP (2005) Critical implication of the (70-96) domain of human immunodeficiency virus type 1 Vpr protein in apoptosis of primary rat cortical and striatal neurons. J Neurovirol 11:489 -502.
20. Everall IP, Luthert PJ, Lantos PL (1993) Neuronal number and volume alterations in the neocortex of HIV infected individuals. J Neurol Neuro-surg Psychiatry 56:481- 486.
21. Fuller RA, Westmoreland SV, Ratai E, Greco JB, Kim JP, Lentz MR, He J, Sehgal PK, Masliah E, Halpern E, Lackner AA, Gonzalez RG (2004) A prospective longitudinal in vivo 1 H MR spectroscopy study of the SIV/ macaque model of neuroAIDS. BMC Neurosci 5:10.
22. Xiao Y, Chen G, Richard J, Rougeau N, Li H, Seidah NG, Cohen EA. Cell-surface processing of extracellular human immunodeficiency virus type 1 Vpr by proprotein convertases Virology 2008. 372: 384-397.

## Claims

1. A method for analyzing the Vpr protein of a HIV, in particular a HIV-1, said Vpr protein being termed "tested Vpr protein", which method comprises: in a biological sample previously obtained from a patient infected with a HIV, and in particular with a HIV-1, performing an analysis of the nucleotide sequence encoding the tested Vpr protein and/or of the amino acid sequence of the tested Vpr protein, in order to determine the amino acid residue of its sequence which is termed X₈₄ and/or the amino acid residue of its sequence which is termed X₈₅, which residues would respectively correspond to the residues at position 84 and 85 in the sequence of a reference HIV Vpr protein.

2. The method according to claim 1, which comprises: in a biological sample previously obtained from a patient infected with a HIV, and in particular with a HIV-1, performing an analysis of the nucleotide sequence encoding the tested Vpr protein and/or of the amino acid sequence of the tested Vpr protein, in order to determine whether amino acid sequence X₈₄X₈₅ consists of sequence isoleucine-glutamine (IQ) or of a peptide sequence chosen from the following group: IQ, TR, IR, LR, IP, LQ, TP, MR, VR, LP, SL and QQ, and in particular from the following group: IQ, TR, IR, LR, IP, LQ, TP, MR, VR and LP.

3. The method according to any one of claims 1 to 3, wherein the analysis of the nucleotide sequence encoding the tested Vpr protein is performed by PCR, using:
- one or several set(s) of primers chosen for their ability to allow amplification of the nucleic acid sequence encoding a region in a Vpr protein which comprises the X₈₄X₈₅ domain; and/or
- one or several set(s) of probes enabling the detection of the amplified products....

4. A method for functionally classifying one or several HIV(s), in particular one or several HIV-1(s), said method comprising analyzing the Vpr protein of said HIV(s), by a method according to any one of claims 1 to 4.

5. A method for providing prognosticating elements, in relation to *in vitro* and/or *ex vivo,* progression or long-term non-progression of a HIV infection or of HIV-associated disorders in a patient, and in particular for providing prognosticating elements, in relation to *in vitro* and/or *ex vivo,* genesis or progression of AIDS and/or neuro-AIDS and in particular of HIV associated dementia (HAD) and tauopathies, said method comprising the following steps:
a) analyzing, by a method according to any one of claims 1 to 4, one or several HIV-Vpr protein(s) which is (are) present in a biological sample previously obtained from a patient infected with at least one HIV, in particular at least one HIV-1; and
b) using the results obtained in step a) in a process for determining possible progression or long-term non-progression of HIV infection or HIV-associated disorders, wherein
- the finding that in a Vpr protein present in the biological sample, X₈₄ is not amino acid residue I and/or that X₈₅ is not amino acid residue Q, is indicative that a long-term non progression of HIV infection or HIV-associated disorders in the patient may be expected, whereas
- the finding that in a Vpr protein present in the biological sample, amino acid sequence X₈₄X₈₅ is isoleucine-glutamine (IQ) is indicative that a progression of HIV infection or HIV-associated disorders in the patient may be expected, and in particular that genesis or progression of AIDS and/or of neuro-AIDS and especially of HAD and tauopathies in the patient may be expected.

6. A method for providing prognosticating elements, in relation to *in vitro* and/or *ex vivo,* progression or long-term non-progression of a HIV infection or of HIV-associated disorders in a patient, and in particular for providing prognosticating elements, in relation to *in vitro* and/or *ex vivo,* genesis or progression of AIDS and/or neuro-AIDS and in particular HIV associated dementia (HAD) and tauopathies, said method comprising the following steps:
a) determining whether a biological sample previously obtained from a patient infected with at least one HIV, in particular at least one HIV-1, contains at least one HIV Vpr protein which is able to bind a PP2A holoenzyme or one of its subunits, in particular PP2A1, or which is able to induce or increase apoptosis of tumor cells or tumor cell lines and especially human tumor cells or cell lines; and
b) using the results obtained in step a) in a process for determining possible progression or long-term non-progression of HIV infection or HIV-associated disorders, wherein
- the presence, in the biological sample, of at least one Vpr protein having said ability is indicative that a progression of HIV infection or HIV-associated disorders in the patient may be expected, and in particular that genesis or progression of AIDS and/or of neuro-AIDS and especially of HAD and tauopathies in the patient may be expected, whereas
- the absence, in the biological sample, of any Vpr protein having said ability is indicative that a long-term non progression of HIV infection or HIV-associated disorders in the patient may be expected.

7. A peptide of less than 30 amino acids in size, preferably less than 20 amino acids, which binds a type 2A Protein Phosphatase (PP2A) holoenzyme or one of its subunits, for example PP2A₁, and which is derived from the C-terminal region of a Vpr protein of a HIV, for example HIV-1, and comprises all or part of the sequence of said Vpr protein which would correspond to the one ranging from amino acid residues 77 to 92 in the sequence of a reference HIV Vpr protein, and which comprises the region of said Vpr protein which would correspond to the one ranging from amino acid residues 84 to 85 in the sequence of said reference HIV Vpr protein, wherein the sequence of said peptide comprises sequence isoleucine-glutamine (IQ), and does not consist of one of the following sequences:
RHSRIGIIQQRRTRNG (SEQ ID No: 11);
RHSRIGIIQQRR (SEQ ID No: 12);
SRIGIIQQRRTR (SEQ ID No: 13); and
IGIIQQRRTRNG (SEQ ID No: 14).

8. The peptide according to claim 8, wherein the reference Vpr protein is the Vpr protein of the Lai HIV-1 isolate or of the HIV-1 89.6 isolate.

9. A peptide which binds a type 2A Protein Phosphatase (PP2A) holoenzyme or one of its subunits, in particular PP2A₁, which is selected from the following group of peptides:
a) a peptide comprising or consisting of a peptide sequence chosen from the following group:
X₇₇HSX₈₀IGIIQQRRX₈₉RNG (SEQ ID No: 3);
X₇₇HSX₈₀IGIIQQRR (SEQ ID No: 4);
SX₈₀IGIIQQRRX₈₉R (SEQ ID No: 5);
IGIIQQRRX₈₉RNG (SEQ ID No: 6);
X₈₀IGIIQQRRX₈₉ (SEQ ID No: 7);
X₈₀IGIIQQRR (SEQ ID No: 8);
IGIIQQRRX₈₉ (SEQ ID No: 9); and
IGIIQQRR (SEQ ID No: 10);
wherein X₇₇, X₈₀ and X₈₉ independently designate one or several amino acid residue(s), which can be, independently, any amino acid residue(s), and
b) a peptide the sequence of which is distinguished from the sequence envisaged in a) by insertion, substitution or deletion of amino 1 to 5 amino acid residues, in particular 1, 2, 3, 4 or 5 amino acid residue(s),
which peptide does not consist of one of the following sequences:
RHSRIGIIQQRRTRNG (SEQ ID No: 11);
RHSRIGIIQQRR (SEQ ID No: 12);
SRIGIIQQRRTR (SEQ ID No: 13); and
IGIIQQRRTRNG (SEQ ID No: 14).

10. The peptide according to any one of claims 8 to 10, which is selected from the following group of peptides:
a) a peptide comprising or consisting of a peptide sequence chosen from the following group:
AHSAIGIIQQRRTRNG (SEQ ID No: 15);
RHSAIGIIQQRRTRNG (SEQ ID No: 16);
AHSRIGIIQQRRTRNG (SEQ ID No: 17);
AHSAIGIIQQRRARNG (SEQ ID No: 18);
RHSAIGIIQQRRARNG (SEQ ID No: 19);
AHSRIGIIQQRRARNG (SEQ ID No: 20)
RHSRIGIIQQRRARNG (SEQ ID No: 21);
AHSAIGIIQQRR (SEQ ID No: 22);
AHSRIGIIQQRR (SEQ ID No: 23);
RHSAIGIIQQRR (SEQ ID No: 24);
SAIGIIQQRRAR (SEQ ID No: 25);
SAIGIIQQRRTR (SEQ ID No: 26);
SRIGIIQQRRAR (SEQ ID No: 27);
IGIIQQRRARNG (SEQ ID No: 28);
AIGIIQQRRA (SEQ ID No: 29);
AIGIIQQRRT (SEQ ID No: 30);
RIGIIQQRRA (SEQ ID No: 31);
AIGIIQQRR (SEQ ID No: 32);
IGIIQQRRA (SEQ ID No: 33);
optionally, RIGIIQQRRT (SEQ ID No: 34); and
optionally, RIGIIQQRR (SEQ ID No: 35); and
optionally, IGIIQQRRT (SEQ ID No: 36); and
optionally, IGIIQQRR; (SEQ ID No: 10); and
b) a peptide, the sequence of which is distinguished from the peptide sequence envisaged in a) by insertion, substitution or deletion of amino 1 to 5 amino acid residues, in particular 1, 2, 3, 4 or 5 amino acid residue(s).

11. A polynucleotide **characterized in that** its sequence comprises or consists of the sequence encoding a peptide according to any one of claims 8 to 10.

12. A method for the preparation of polyclonal or monoclonal antibodies, which is **characterized in that** a peptide as recited in any one of claims 8 to 11 is used as an antigen to elicit antibodies production.

13. Use of a polynucleotide according to claim 12 or antibodes prepared by the method according to claim12, in the *in vitro* diagnosis of a HIV infection.

14. A method of screening one or several peptide(s) derived from the Vpr protein of one or several HIV isolate(s) in particular HIV-1 isolate(s), for
a) its (their) ability to bind a type 2A Protein Phosphatase (PP2A) holoenzyme or one of its subunits, in particular PP2A₁, and/or
b) its (their) ability to induce or increase apoptosis of tumor cells or tumor cell lines, especially human tumor cells or cell lines, and/or
c) its (their) ability to deliver a compound, in particular a peptide or polypeptide, into a cell,
said method comprising
1) depositing, on a support, peptides the sequence of which is derived from the C-terminal region of the Vpr protein of one or several HIV(s) in particular HIV-1(s), which peptides comprise amino acid sequence IQ and are as defined in any one of claim 8 or 9;
2) bringing the support into contact with a solution containing the PP2A holoenzyme or one of its subunits under conditions that allow the peptides present on the support to bind the PP2A holoenzyme or one of its subunits; and
3) identifying, on the support, the peptide(s) to which the PP2A holoenzyme or one of its subunits is bound.

15. An element selected from a peptide according to one of claims 8 to 12, a polynucleotide according to claim 13, antibodies prepared by the method according to claim 14, or from Vpr-derived peptides screened by the method according to claim 15, for use for delivering one or several compounds in particular one or several polypeptides into a cell, and/or for use for inducing or increasing apoptosis of tumor cells or tumor cell lines, especially human tumor cells or cell lines and more generally human cells.

16. A method of screening a test compound for its ability to interfere with the binding of a HIV Vpr protein or of one or several peptide(s) or polypeptide(s) derived from a HIV Vpr protein, to a type 2A Protein Phosphatase (PP2A) holoenzyme or to one of its subunits, which peptides or polypeptides comprise the amino acid residues of the Vpr protein which would respectively correspond to the residues at position 84 and 85 in the sequence of a reference HIV Vpr protein, said method comprising or consisting of the steps of:
a) providing an assay system comprising:
- a Vpr protein of a HIV, in particular a Vpr protein of a HIV-1, or a fragment of said Vpr protein, said fragment comprising the amino acid residues of said Vpr protein which would respectively correspond to the residues at position 84 and 85 in the sequence of a reference HIV Vpr protein, and said fragment retaining the ability of said Vpr protein to bind a PP2A holoenzyme or one of its subunits; and
- a PP2A holoenzyme or one of its subunits, for example PP2A_{1;}
b) contacting said assay system with the test compound in conditions enabling the interaction between the test compound and the PP2A holoenzyme or one of its subunits;
c) determining whether said test compound interferes with the binding of the Vpr protein or a fragment thereof to the PP2A holoenzyme or to one of its subunits.
